19 Europäisches Patentamt
European Patent Office
Office européen des brevets

11 Veröffentlichungsnummer: **0 081 461**
**A2**

12 # EUROPÄISCHE PATENTANMELDUNG

21 Anmeldenummer: 82810516.3

22 Anmeldetag: 01.12.82

51 Int. Cl.³: C 07 D 487/04
A 61 K 31/505
//(C07D487/04, 243/00, 235/00)

30 Priorität: 07.12.81 US 328274

43 Veröffentlichungstag der Anmeldung:
15.06.83 Patentblatt 83/24

84 Benannte Vertragsstaaten:
AT BE CH DE FR IT LI LU NL SE

71 Anmelder: CIBA-GEIGY AG
Patentabteilung Postfach
CH-4002 Basel(CH)

72 Erfinder: Vlattas, Isidoros, Dr.
131 Butler Parkway
Summit New Jersey 07901(US)

54 **Imidazobenzodiazepine, Verfahren zu ihrer Herstellung, pharmazeutische Präparate enthaltend diese Verbindungen, sowie ihre therapeutische Verwendung.**


57 Die Erfindung betrifft psychoaktive und antiallergische Verbindungen der Formel I

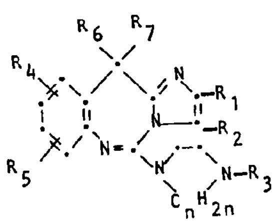

(I),

worin jedes der Symbole $R_1$ und $R_2$ Wasserstoff, Niederalkyl, Niederalkanoyl, Halogen, Cyan, Carboxy, Niederalkoxycarbonyl, Carbamoyl, Sulfamoyl, Mono- oder Di-niederalkyl-(carbamoyl oder -sulfamoyl) bedeutet, $C_nH_{2n}$ für Niederalkylen steht, welches die zwei Stickstoffatome durch 2 oder 3 Kohlenstoffatome trennt, und $R_3$ für Wasserstoff, Niederalkyl, Niederalkenyl, Niederalkynyl, Niederalkanoyl, Arylniederalkyl, Niederalkoxycarbonyl, Phenylniederalkoxycarbonyl, oder (Hydroxy Niederalkanoyloxy, Aryloxy oder Niederalkoxy)-niederalkyl steht worin der Niederalkylrest mindestens 2 Kohlenstoffatome enthält $R_4$ und $R_5$ unabhängig voneinander Wasserstoff, Niederalkyl, Niederalkoxy, Niederalkylthio, Halogen, Trifluormethyl, Hydroxy, Niederalkanoyloxy, Sulfamoyl, Mono- oder Diniederalkylsulfamoyl, und $R_6$ und $R_7$ Wasserstoff oder Niederalkyl bedeuten; ihre N-Oxide, ihre Niederalkyl quaternären Derivate, und Salze, insbesondere therapeutisch verwendbaren Salze mit Säuren oder Basen, von allen diesen Verbindungen. Sie können z.B. durch Kondensation einer Verbindung der Formel III

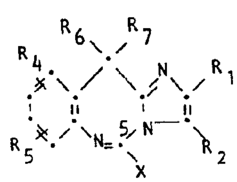

(III),

worin X eine zusammen mit Wasserstoff oder einem Alkalimetall abspaltbare Gruppe bedeutet, mit einer Verbindung der Formel IV

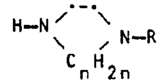

(IV)

oder einem Alkalimetallderivat davon, hergestellt werden


EP 0 081 461 A2

CIBA-GEIGY AG
Basel (Schweiz)

4-13694/+

Imidazobenzodiazepine, Verfahren zu ihrer Herstellung, pharmazeutische Präparate enthaltend diese Verbindungen, sowie ihre therapeutische Verwendung

Die Erfindung betrifft neue 5-Diazacycloalkylimidazo[1,2-c][1,3]-benzodiazepine der allgemeinen Formel I

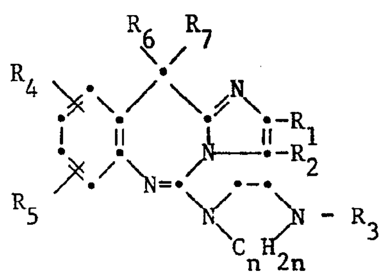

(I) ,

worin jedes der Symbole $R_1$ und $R_2$ Wasserstoff, Niederalkyl, Niederalkanoyl, Halogen, Cyan, Carboxy, Niederalkoxycarbonyl, Carbamoyl, Sulfamoyl, Mono- oder Di-niederalkyl-(carbamoyl oder -sulfamoyl) bedeutet, $C_nH_{2n}$ für Niederalkylen steht, welches die zwei Stickstoffatome durch 2 oder 3 Kohlenstoffatome trennt, und $R_3$ für Wasserstoff, Niederalkyl, Niederalkenyl, Niederalkynyl, Niederalkanoyl, Arylniederalkyl, Niederalkoxycarbonyl, Phenylniederalkoxycarbonyl, oder (Hydroxy, Niederalkanoyloxy, Aryloxy oder Niederalkoxy)-niederalkyl steht, worin der Niederalkylrest mindestens 2 Kohlenstoffatome enthält, $R_4$ und $R_5$ unabhängig voneinander Wasserstoff, Niederalkyl, Niederalkoxy, Niederalkylthio, Halogen, Trifluormethyl, Hydroxy, Niederalkanoyloxy, Sulfamoyl, Mono- oder Diniederalkylsulfamoyl, und $R_6$ und $R_7$ Wasserstoff oder Niederalkyl bedeuten; ihre N-Oxide, ihre Niederalkyl quaternären Derivate, und Salze, insbesondere therapeutisch verwendbaren Salze mit Säuren oder Basen, von allen diesen Ver-

BAD ORIGINAL

bindungen, sowie Verfahren zu ihrer Herstellung, pharmazeutische Präparate enthaltend diese Verbindungen, sowie ihre therapeutische Verwendung.

Der Ausdruck "nieder" definiert in den oben oder nachfolgend genannten organischen Resten oder Verbindungen, wie z.B. Alkyl, Alkenyl und Alkynyl, solche mit höchstens 7, vorzugsweise 4, insbesondere 1 oder 2 Kohlenstoffatomen.

Ein Halogenatom ist vorzugsweise Fluor oder Chlor, aber auch Brom oder Jod.

Eine Niederalkylgruppe oder eine solche in den genannten Alkoxy-, Alkylthio- oder anderen genannten alkylierten Gruppen, ist vor allem Methyl und auch Aethyl, n- oder iso-(Propyl, Butyl, Pentyl, Hexyl oder Heptyl), z.B. 2-Methylpropyl oder 3-Methylbutyl, Niederalkenyl bedeutet vorzugsweise Allyl und Niederalkynyl vorzugsweise Propargyl.

Arylniederalkyl bedeutet vorzugsweise Benzyl, 1-, 2- oder 3-Phenylpropyl, 1- oder 2-Phenyläthyl. Solche Substituenten können im Phenylring gegebenenfalls durch Halogen, Niederalkoxy oder Niederalkyl substituiert sein.

Eine Niederalkoxygruppe enthält vorzugsweise 1 bis 4 Kohlenstoffatome und bedeutet beispielsweise Aethoxy, Propoxy, Isopropoxy oder insbesondere jedoch Methoxy.

Eine Niederalkylthiogruppe enthält vorzugsweise 1 bis 4 Kohlenstoffatome und bedeutet beispielsweise Aethylthio, Propylthio oder insbesondere Methylthio.

Eine Acylgruppe bedeutet beispielsweise Niederalkanoyl, Niederalkoxycarbonyl, Carbamoyl, Sulfamoyl, Mono- oder Di-niederalkyl-(carbamoyl oder sulfamoyl), Halosulfonyl, oder Phenylniederalkoxycarbonyl und ähnliche.

Niederalkanoyl steht vorzugsweise für Acetyl oder Propionyl und Niederalkanoyloxy vorzugsweise für Acetyloxy oder Propionyloxy.

Eine Niederalkoxycarbonyl-, Mono- oder Di-niederalkylcarbamoyl- oder Mono- oder Di-niederalkylsulfamoylgruppe ist vorzugsweise Methoxycarbonyl, Aethoxycarbonyl; Mono- oder Dimethylcarbamoyl bzw. Mono- oder Dimethylsulfamoyl.

Eine Phenylniederalkoxycarbonylgruppe ist z.B. Phenylmethoxycarbonyl oder Phenyläthoxycarbonyl.

Eine Niederalkylengruppe $C_nH_{2n}$ ist insbesondere Aethylen, aber auch 1,2- oder 1,3-Propylen, 1,2-, 1,3- oder 2,3-Butylen, welche mit den benachbarten Stickstoffatomen vorzugsweise eine Piperazino- oder Homopiperazinogruppe bilden.

Eine Hydroxyniederalkylgruppe ist vorzugsweise 2-Hydroxy-(äthyl oder propyl), 3-Hydroxy-(propyl oder butyl) bzw. 4-Hydroxybutyl.

Eine Niederalkanoyloxyniederalkylgruppe bedeutet vorzugsweise Niederalkanoyloxy-(äthyl, propyl oder butyl), beispielsweise 2-Acetyloxy- oder 2-Propionyloxy- (äthyl, propyl oder butyl), 3-Acetyloxy- oder 3-Propionyloxy-(propyl oder butyl), 4-Acetyloxy- oder 4-Propionyloxybutyl und ähnlich zusammengesetzte Gruppen.

Eine Niederalkoxyniederalkylgruppe bedeutet vorzugsweise Niederalkoxy-(äthyl, propyl oder butyl), wie z.B. 2-Methoxy- oder Aethoxy-(äthyl, propyl oder butyl), 3-Methoxy- oder 3-Aethoxy(propyl oder butyl), 4-Methoxy- oder 4-Aethoxybutyl und ähnlich zusammengesetzte Gruppen.

Eine Aryloxyniederalkylgruppe bedeutet vorzugsweise Phenyloxy-(äthyl, propyl oder butyl), wobei solche Gruppen gegebenenfalls im Phenylring vorzugsweise durch Halogen, Niederalkoxy oder auch Niederalkyl substituiert sein können.

BAD ORIGINAL

Niederalkyl quaternäre Derivate von Verbindungen der Formel I sind vorzugsweise z.B. Methyl, Aethyl oder Propyl quaternäre Salze abgeleitet von reaktionsfähigen Estern niederer Alkanole mit vorzugsweise 1 bis 4 Kohlenstoffatomen, wie z.B. Methanol, Aethanol oder Propanol. Die Anionen der erwähnten Derivate, d.h. Salze sind vorzugsweise solche, welche den therapeutisch verwendbaren Säuren entsprechen, wie z.B. Halogenide, beispielsweise Bromide oder Jodide, Sulfat, oder Niederalkylsulfonate, wie z.B. Methylsulfonat.

Obwohl die N-Oxide oder Niederalkyl quaternären Salze von Verbindungen der Formel I auch solche betreffen, in welchem ein oder auch mehrere Ringstickstoffatome funktionalisiert sind, werden die besagten N-Oxide, Niederalkyl-quaternären Salze vorzugsweise von Verbindungen der Formel I abgeleitet, in welchen $R_3$ Niederalkyl, Aryl-niederalkyl, oder (Hydroxy-, Niederalkanoyloxy-, Aryloxy- oder Niederalkoxy-)niederalkyl, mit mindestens 2 Kohlenstoffatomen im Niederalkylrest, bedeuten und in welchen das Stickstoffatom, welches den Substituenten $R_3$ trägt, funktionalisiert ist.

Diese besagten Verbindungen der Formel I ergeben Säureadditionssalze, vorzugsweise mit therapeutisch verwendbaren anorganischen oder auch organischen Säuren, wie z.B. mit starken Mineralsäuren, wie Halogenwasserstoffsäuren, z.B. Chlorwasserstoff- oder Bromwasserstoffsäure, Schwefel-, Phosphor oder auch Salpetersäure, oder mit organischen Säuren, wie z.B. aliphatischen oder aromatischen Carbon- oder Sulfonsäuren, z.B. Ameisen-, Essig-, Propion-, Bernstein-, Glykol-, Milch-, Aepfel-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl, Pamoe-, Nikotin-, Methansulfon-, Aethansulfon-, Hydroxyäthansulfon-, Aethylensulfon-, Halogenbenzolsulfon-, Toluolsulfon-, Naphthalinsulfon-, Sulfanil- oder Cyclohexylsulfaminsäure; oder die Ascorbinsäure.

Die Verbindungen der Formel I, in welchen $R_1$ und/oder $R_2$ Carboxy bedeutet, bilden auch Salze mit Basen, vorzugsweise solchen, welche therapeutisch verwendbare Salze ergeben, z.B. Ammoniak, Mono-, Di- oder Tri-niederalkylaminen, Niederalkylenaminen, Morpholin, Piperazin, Pyridin oder mit Niederalkylderivaten der genannten cyclischen Basen; Alkalimetall- oder Erdalkalimetallhydroxyden.

Die Verbindungen der Erfindung zeigen wertvolle pharmakologische Eigenschaften, insbesondere psychoaktive, wie z.B. neuroleptische Wirkungen, aber auch antiallergische, wie z.B. antihistaminische Wirkungen. Diese können in Tierversuchen, vorzugswiese an Säugern, z.B. Mäusen, Ratten, Meerschweinchen oder Affen, als Testobjekte, nachgewiesen werden. Die genannten Verbindungen können ihnen enteral oder parenteral, vorzugsweise oral oder subkutan, intravenös oder intraperitoneal, z.B. durch Steckkapseln oder in Form von Stärke enthaltenden Suspensionen bzw. wässerigen Lösungen, verabreicht werden. Die verwendete Dosis kann in einem Bereich von ungefähr zwischen 0,1 und 100 mg/kg/Tag, vorzugsweise ungefähr 0,5 und 50 mg/kg/Tag, insbesondere zwischen 1 und 30 mg/kg/Tag liegen.

Die genannten neuroleptischen Eigenschaften können an ausgewachsenen Ratten oder Totenkopf-Aeffchen (squirrel monkeys) nachgewiesen werden. Die Tiere sind zur Betätigung eines Hebels trainiert, wodurch sie einem auf den Fuss verabreichten elektrischen Schock ausweichen können. Jeder Hebeldruck verschiebt den Schock um 30 Sekunden. Vergisst das Tier den Hebel innerhalb des genannten Zeitintervalls einmal zu drücken, so werden alle 15 Sekunden kurze (0,5 sec.) elektrische Schocks abgegeben, bis das Tier den Hebel wieder betätigt. Unter Kontrollbedingungen drücken die Versuchstiere den Hebel mit einer mässig ausgeglichenen Geschwindigkeit und erhalten selten mehr als 5 oder 6 Schocks während einer 25-minuten (Ratten) bis 4-stündigen Versuchsperiode. Die genannten Verbindungen, welche den Tieren 30, 90 und 210 Minuten vor dem Versuch verabreicht werden, blockieren das angelernte konditionierte Vermeidungs-Verhalten.

BAD ORIGINAL

Die Folge ist die Abnahme der Vermeidungs-Reaktion und eine wesentliche Zunahme der von den Tieren erlittenen Schocks. Sowohl die Anzahl der Vermeidungs-Reaktionen als auch diejenige der Fehlverhalten (der erhaltenen Schocks) werden zur Auswertung registriert.

Schliesslich können die antihistaminischen Eigenschaften in vitro gemäss Chasin et al., J. Neurochem. 22, 1031 (1974) nachgewiesen werden. Homogenate eines zellfreien Präparats der Grosshirnrinde von Meerschweinchen werden vorher mit $^{3}H$-Adenin inkubiert, wobei endogenes $^{3}H$-Adenosin-triphosphat gebildet wird. Die Homogenate werden dann mit 50 mikromolarem Histamin inkubiert, um die Synthese von $^{3}H$-cyclischem Adenosinmonophosphat zu aktivieren. Dieses Vorgehen wird in Gegenwart oder Abwesenheit der Testverbindung unternommen, wobei man Konzentrationen zwischen 0,01 und 100 Mikromolen einsetzt. Ist die geprüfte Substanz aktiv, so wird die Histamin-Aktivierung der Adenylat-Cyclase gehemmt. Der $IC_{50}$-Wert bedeutet die Konzentration, bei welcher die Histamin-Aktivierung um 50% gehemmt wird.

Die erfindungsgemässen Verbindungen können dementsprechend als Neuroleptika und Antihistaminpräparate, z.B. in der Behandlung und Handhabung von psychotischen Erscheinungen wie z.B. Aggressions-, Agitations-, Schizophrenieerscheinungen und/oder von allergischen Zuständen von Säugern, inklusive Menschen, verwendet werden. Sie können auch als Zwischenprodukte zur Herstellung von anderen wertvollen Produkten, insbesondere von pharmakologisch wirksamen Präparaten eingesetzt werden.

Bevorzugte erfindungsgemässe Verbindungen sind diejenigen der Formel I, worin jedes der Symbole $R_1$ und $R_2$ Wasserstoff, Niederalkyl, Cyan, Carboxy, Niederalkoxycarbonyl oder Carbamoyl, und n die ganze Zahl 2-4 bedeuten, $R_3$ für Wasserstoff, Niederalkyl, Niederalkoxycarbonyl oder Hydroxy-niederalkyl mit 2-4 Kohlenstoffatomen im Niederalkylrest steht, $R_4$ Wasserstoff, Niederalkyl, Niederalkoxy, Niederalkylthio, Halogen oder Trifluormethyl bedeutet, und $R_5$ für

BAD ORIGINAL

Wasserstoff steht, und $R_6$ und $R_7$ die Bedeutung von Wasserstoff oder Niederalkyl haben, ihre N-Oxide, Niederalkyl quaternären Derivate, und Salze, insbesondere therapeutisch verwendbaren Salze, von allen diesen Verbindungen.

Bevorzugt sind weiter Verbindungen der Formel I, worin jedes der Symbole $R_1$ und $R_2$ Wasserstoff, Methyl, Aethyl, Cyano, Carboxy, Niederalkoxycarbonyl mit 1-3 Kohlenstoffatomen im Niederalkoxyrest oder Carbamoyl bedeutet, n eine ganze Zahl 2 oder 3 bedeutet, $R_3$ für Wasserstoff, Niederalkyl mit 1-3 Kohlenstoffatomen, Niederalkoxycarbonyl mit 1-3 Kohlenstoffatomen im Niederalkoxyrest, Hydroxyäthyl oder Hydroxypropyl steht, $R_4$ Wasserstoff, Methyl, Methoxy, Methylthio, Chlor oder Trifluormethyl, $R_5$ Wasserstoff und $R_6$ und $R_7$ Wasserstoff oder Methyl bedeuten, ihre N-Oxide, Methyl quaternären Derivate, und Salze, insbesondere therapeutisch verwendbaren Salze, von allen diesen Verbindungen.

Besonders hervorzuheben sind Verbindungen der Formel II

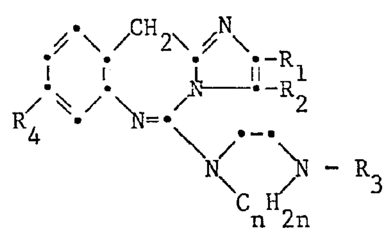

(II) ,

worin jedes der Symbole $R_1$ und $R_2$ unabhängig voneinander Wasserstoff oder Niederalkyl bedeutet, $R_3$ für Wasserstoff, Niederalkyl oder Hydroxy-niederalkyl steht, worin Hydroxy vom Stickstoffatom durch mindestens 2 Kohlenstoffatome getrennt ist, $R_4$ Wasserstoff, Niederalkyl, Niederalkoxy, Niederalkylthio, Halogen oder Trifluormethyl bedeutet, $C_nH_{2n}$ für Aethylen oder Propylen steht, ihre N-Oxide und Salze, insbesondere therapeutisch verwendbaren Salze davon.

Von besonderem Interesse sind diejenigen Verbindungen der Formel II, worin jedes der Symbole $R_1$ und $R_2$ unabhängig voneinander Wasserstoff oder Methyl, $R_3$ Wasserstoff, Methyl, Aethyl, Propyl, 2-Hydroxyäthyl

oder 3-Hydroxy-propyl bedeuten, und $R_4$ für Wasserstoff, Methyl, Methoxy, Fluor, Chlor oder Trifluormethyl steht, $C_nH_{2n}$ Aethylen oder Propylen bedeutet, ihre N-Oxide und Salze, insbesondere therapeutisch verwendbaren Salze davon.

Des weiteren von besonderer Bedeutung sind Verbindungen der Formel II, worin $R_1$ und $R_2$ unabhängig voneinander Wasserstoff oder Methyl, und $R_3$ Wasserstoff, Methyl, Aethyl, Propyl, 2-Hydroxyäthyl bedeuten, und $R_4$ für Wasserstoff, Methyl, Fluor, Chlor oder Trifluormethyl steht und $C_nH_{2n}$ Aethylen bedeutet, und ihre Salze, insbesondere therapeutisch verwendbaren Salze.

Besonders als Vertreter der erfindungsgemässen Verbindungen weist die Verbindung des nachfolgenden Beispiels 1, das 5-(4-Methyl-1-piperazinyl)-11H-imidazo[1,2-c][1,3]benzodiazepin auf die antipsychotischen Eigenschaften hin. Die Verbindung vermindert die Vermeidungs-Reaktionen (Zunahme der Fehlverhalten) bei Ratten und Totenkopf-Aeffchen bei einer oral verabreichten Gesamtdosis von 30 mg/kg oder darunter.

Die anti-histaminische Wirkung wird insbesondere von 5-(4-Methyl-1-piperazinyl)-11H-imidazo[1,2-c][1,3]benzodiazepin (Verbindung des Beispiels 1) veranschaulicht. Diese Verbindung hemmt die Histaminaktivierung der Adenylat-Cyclase mit einem $IC_{50}$ von ungefähr $1 \times 10^{-6}$ Mol.

Ferner sei noch zu erwähnen, dass die Verbindung des nachfolgenden Beispiels 1, das 5-(4-Methyl-1-piperazinyl)-11H-imidazo[1,2-c][1,3]-benzodiazepin sich auch dadurch auszeichnet, dass sie im wesentlichen frei von extrapyramidalen Nebenwirkungen ist, wie z.B. Diskinesien und Dystonien in Totenkopf-Aeffchen (dyskinetische Bewegungen und dystonische Körperhaltungen), und lediglich eine minimale α-adrenergische Blockierungsaktivität <u>in vitro</u> aufweist.

Die Verbindungen der Erfindungen werden nach an sich bekannten Methoden, z.B. dadurch hergestellt, dass man

a) eine Verbindung der Formel III

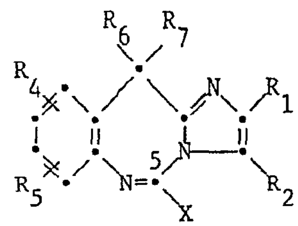

(III) ,

worin X eine zusammen mit Wasserstoff oder einem Alkalimetall abspaltbare Gruppe bedeutet und die anderen Symbole die unter der Formel I angegebenen Bedeutungen haben, mit einer Verbindung der Formel IV

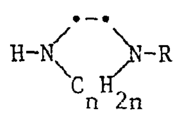

(IV)

oder einem Alkalimetallderivat davon, in welcher $R_3$ die unter der Formel I angegebene Bedeutung hat, kondensiert, und, wenn erwünscht, eine erhaltene Verbindung der Formel I in eine andere der Erfindung umwandelt.

Eine zusammen mit Wasserstoff oder einem Alkalimetallatom abspaltbare Gruppe ist z.B. in erster Linie eine freie oder vorzugsweise verätherte Mercaptogruppe, ferner eine gegebenenfalls funktionell abgewandelte reaktionsfähige Hydroxygruppe, die Cyanato- oder Thiocyanatogruppe oder die Nitroaminogruppe. Eine verätherte Mercaptogruppe ist in erster Linie eine durch einen gegebenenfalls substituierten Kohlenwasserstoffrest, insbesondere aliphatischen Charakters, verätherte Mercaptogruppe. Sie stellt in erster Linie Niederalkylthio, z.B. Methylthio, Aethylthio oder Butylthio oder Phenylniederalkylthio, z.B. Phenylthio oder Benzylthio dar. Eine gegebenenfalls funktionell abgewandelte reaktionsfähige Hydroxygruppe ist eine freie und u.a. eine entsprechende veresterte Hydroxygruppe. Eine solche ist u.a. Halogen, z.B. Chlor oder Brom, oder Niederalkylsulfonyloxy, z.B. Methan-

BAD ORIGINAL

sulfonyloxy. Eine verätherte Hydroxygruppe ist beispielsweise eine Niederalkoxygruppe, wie z.B. Methoxy oder Aethoxy.

Diese Kondensation erfolgt vorteilhafterweise in einem Ueberschuss der eingesetzten Verbindung der Formel IV oder mit einer äquivalenten Menge der in situ hergestellten Alkalimetallverbindung davon, wenn X als abspaltbare Gruppe einer Verbindung der Formel III vorzugsweise Halogen, Niederalkylthio oder Thiocyanato bedeutet. Die Umsetzung erfolgt je nach der Bedeutung von X bei Temperaturen zwischen 0° und 150°C, vorzugsweise in einem geeigneten Lösungsmittel, wie z.B. Niederalkanol, beispielsweise, Amylalkohol, Dimethylformamid, Hexamethylphosphoramid oder Toluol. Die besagte Kondensation einer Verbindung der Formel III mit einer Verbindung der Formel IV kann jedoch auch in Gegenwart einer Säure, beispielsweise einer Halogenwasserstoffsäure, wie z.B. Chlorwasserstoff (Salzsäure) ausgeführt werden.

Die neuen 11H-Imidazo[1,2-c][1,3]benzodiazepin-Ausgangsstoffe der Formel III werden nach an sich bekannten Ringschluss-Methoden hergestellt. Vorteilhafterweise kondensiert man Verbindungen der Formel V

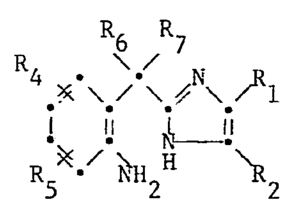

(V)

worin $R_1$, $R_2$, $R_4$-$R_7$ die unter der Formel I angegebenen Bedeutungen haben, mit reaktionsfähigen Kohlensäure-Derivaten, wie z.B. Phosgen, Thiophosgen, 1,1'-Carbonyldiimidazol, Bromcyan oder Chlorameisensäurephenylester.

Verbindungen der Formel III, in welchen X Hydroxy bedeutet, können in solche, worin X für Sulfhydryl steht, mit konventionellen Sulfurierungsmitteln, z.B. mit Phosphorpentasulfid umgewandelt werden.

BAD ORIGINAL

Diese können in Verbindungen, in welchen X die obige, unter Formel III angegebene Bedeutung hat, analog zu den in den Beispielen beschriebenen Methoden übergeführt werden.

Ausgangsverbindungen der Formel V werden vorzugsweise durch Reduktion der entsprechend verschieden substituierten 2-(o-Nitrobenzyl)-imidazolen durch Reduktion hergestellt werden, die wieder vorzugsweise aus den entsprechend substituierten o-Nitrobenzylnitrilen und 2-Aminoacetalen (oder Ketalen), beispielsweise Aminoacetaldehyddimethylacetal, nach bekannten, analog zu der in den Beispielen beschriebenen Verfahren hergestellt werden.

Ein weiteres Verfahren zur Herstellung von Verbindungen der allgemeinen Formel I besteht darin, dass man

b) eine Verbindung der Formel VI

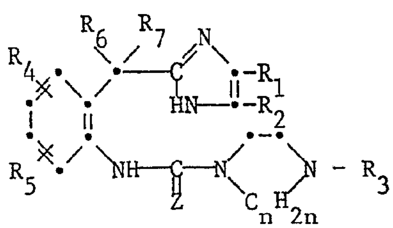

(VI)

unter entwässernden, dehydrosulfurierenden und desaminierenden Bedingungen ringschliesst, worin Z ein Sauerstoff- oder Schwefelatom oder NH bedeutet, und die anderen Symbole die oben angegebenen Bedeutungen haben, und, wenn erwünscht, eine erhaltene Verbindung in eine andere Verbindung der Erfindung umwandelt.

Der genannte Ringschluss wird vorzugsweise bei einer Temperatur zwischen 0° und 120°, beispielsweise mit Phosphorhalogeniden, wie z.B. Phosphorpentachlorid, und/oder -oxyhalogeniden oder Cyanhalogeniden, mit oder ohne Kronenätherkatalysator, z.B. 8-Kronen-6-äther, in Gegenwart oder Abwesenheit von basischen Katalysatoren, wie

BAD ORIGINAL

z.B. Triäthylamin oder Kaliumcarbonat, vorzugsweise in einem inerten Lösungsmittel, z.B. Acetonitril oder Toluol durchgeführt.

Die Ausgangsstoffe der Formel VI können falls sie neu sind, nach an sich bekannten Methoden erhalten werden. Beispielsweise können die Ausgangsstoffe der Formel VI, ausgehend von den (tautomeren) Vorstufen Verbindungen der Formel III, worin X Hydroxy, Thio oder Amino bedeutet, hergestellt werden. Diese Verbindungen werden mit Verbindungen der Formel IV in Gegenwart oder Abwesenheit von anderen Basen, z.B. den oben genannten, vorzugsweise in inerten Lösungsmitteln, wie Methylenchlorid oder Toluol, bei Temperaturen. zwischen 0° und 150°C, vorteilhafterweise zwischen 10° und 50°, kondensiert. Die Ringöffnung wird vorzugsweise bei niedrigen Temperaturen durchgeführt, um Nebenreaktionen der gegebenenfalls vorhandenen reaktionsfähigen funktionellen Gruppen $R_1$ und $R_2$ vorzubeugen.

Auf einem anderen Wege können Ausgangsstoffe der Formel VI, in welchen $R_3$ Niederalkanoyl, Niederalkoxycarbonyl oder Phenyl-niederalkoxycarbonyl bedeutet, durch Kondensation von Verbindungen der Formel V mit einer Verbindung der Formel VII

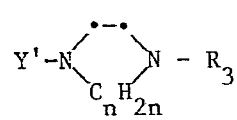

(VII)

worin Y' Halogencarbonyl, Halogenthiocarbonyl oder Cyan bedeuten, vorzugsweise in einem inerten Lösungsmittel wie bereits oben angegeben, bei Temperaturen zwischen 0° und 150°C in Anwesenheit oder Abwesenheit eines basischen Katalysators, wie z.B. Triäthylamin oder Kaliumcarbonat, hergestellt werden.

Ausgangsverbindungen der Formel VII werden vorzugsweise aus Verbindungen der Formel IV, worin $R_3$ die unter der Formel VII angegebenen Bedeutungen hat, oder aus den entsprechenden N-Trimethylsilylderivaten durch Umsetzung mit Phosgen, Thiophosgen oder Bromcyan

in einem inerten Lösungsmittel, wie z.B. Diäthyläther, Methylenchlorid oder Dimethoxyäthan bei Temperaturen zwischen -70°C und +50°C erhalten. Die Umsetzungen können in An- oder auch Abwesenheit eines basischen Katalysators, beispielsweise Triäthylamin oder Kaliumcarbonat, ausgeführt werden.

Die erhaltenen Verbindungen der Erfindung können in an sich bekannter Weise in andere Verbindungen der Erfindungen gemäss Formel I übergeführt werden. So können z.B. Verbindungen, in welchen $R_3$ Wasserstoff oder ein Alkalimetallatom bedeutet, z.B. ihre Natrium- oder Lithiumsalze, mit substituierten oder unsubstituierten Oxiranen, z.B. mit Aethylenoxid, oder mit reaktionsfähigen Estern von unsubstituierten oder entsprechend substituierten aliphatischen oder araliphatischen Alkoholen, z.B. Methanol, Aethanol, Methoxyäthanol, Phenoxyäthanol, Allyl- oder Propargylalkohol, in die entsprechenden N-substituierten Verbindungen oder quaternären Derivate, je nach der molaren Menge des verwendeten Alkylierungsmittel, übergeführt werden. Diese Ester sind beispielsweise von starken anorganischen oder organischen Säuren, vor allem von Halogenwasserstoffsäuren, z.B. Chlorwasserstoff-, Bromwasserstoff- oder Jodwasserstoffsäure, Schwefelsäure oder einer aromatischen Sulfonsäure, z.B. p-Toluolsulfonsäure oder m-Brombenzolsulfonsäure abgeleitet. Zwischenprodukte der Formel 1, in welchen $R_3$ ein Alkalimetallatom bedeutet oder auch Alkalimetallderivate von Verbindungen der Formel IV können durch Metallisierung mit reaktionsfähigen organischen Metallverbindungen, z.B. Lithiumdiisopropylamid, mit Alkalimetall-alkoxiden, z.B. Natrium-methoxid (Natriummethylat) oder mit Alkalimetallhydriden, z.B. Natrium- oder Kaliumhydrid, erhalten werden.

Ungesättigte Verbindungen, z.B. solche, in welchen $R_3$ Niederalkenyl oder Niederalkynyl bedeutet, können mit katalytisch aktiviertem Wasserstoff in die Verbindungen, in welchen $R_3$ den entsprechenden Niederalkylrest bedeutet, umgewandelt werden. Umgekehrt können erhaltene N-alkylierte Verbindungen in N-unsubstituierte Verbindungen

BAD ORIGINAL

umgewandelt werden. Man hydrogenolysiert katalytisch die N-Benzyl-Verbindungen oder man setzt sie z.B. mit Halogenameisensäure-niederalkylestern, z.B. Chlorameisensäure-äthylester um, wobei man N-Acylderivate erhält, welche z.B. mit wässerigen Basen, z.B. Alkalimetallhydroxyden, wie eine wässerige Natriumhydroxydlösung, in die genannten N-unsubstituierten Verbindungen ($R_3$ = H) übergeführt werden können.

Verbindungen der Formel I, in welchen $R_3$ Hydroxyniederalkyl bedeutet, können auch dadurch hergestellt werden, dass man entsprechende Verbindungen der Formel I, in welchen $R_3$ Wasserstoff bedeutet, zuerst mit reaktionsfähigen Derivaten von entsprechenden Glykolen, Glykolsäuren oder Dicarbonsäuren, z.B. ihren Niederalkylestern, Halogeniden der Anhydriden, oder mit reaktionsfähigen Estern der genannten Glykole oder Glykolsäurederivate, z.B. mit Estern von Halogenwasserstoffsäuren oder aromatischen Sulfonsäuren, 1,2-Dibromäthan oder -1,2-Dibrompropan, Bromessigsäure-äthylester oder Brompropionsäureäthylester, Tosyloxyessigsäure-äthylester, Oxalsäurediäthylester, Malonsäurediäthylester oder Oxalsäuremonoäthylester-chlorid umsetzt. Die erhaltenen Zwischenprodukte werden entweder hydrolysiert oder mit einfachen oder komplexen Leichtmetallhydriden, z.B. Lithiumaluminiumhydrid, allein oder mit Diboran, zu den Verbindungen der Formel I, in welchen $R_3$ Hydroxyniederalkyl bedeutet, reduziert.

Verbindungen der Formel I, in welchen $R_3$ Niederalkyl, beispielsweise Methyl, bedeutet, können wie folgt beschrieben,hergestellt werden: Anfangs werden Verbindungen, in welchen $R_3$ für Wasserstoff steht, durch Umsetzung mit Halogenameisensäure-niederalkylestern oder -phenylniederalkylestern, z.B. Chlorameisensäure-äthylester, in die Verbindungen der Formel I, worin $R_3$ Niederalkoxycarbonyl, oder Phenylniederalkoxycarbonyl bedeutet, übergeführt. Dann reduziert man diese Acylderivate mit einfachen oder komplexen Leichtmetallhydriden, z.B. mit Lithiumaluminiumhydrid, Natrium-tri-tert.butoxy-aluminiumhydrid oder Natrium-bis-(2-methoxy-äthoxy)-aluminiumhydrid.

N-Acylderivate, in welchen $R_3$ beispielsweise eine Niederalkanoylgruppe bedeutet, können aus Verbindungen der Formel I, in welchen $R_3$ Wasserstoff bedeutet und entsprechenden reaktionsfähigen Säurederivaten, z.B. Säurehalogeniden, einfachen oder aktivierten Estern, z.B. Alkylestern oder Cyanalkylestern, Anhydriden oder Isocyanaten, erhalten werden. Diese erhaltenen Verbindungen können wiederum in Verbindungen der Formel I, worin $R_3$ Niederalkyl bedeutet, wie oben angegeben reduziert werden, Verbindungen der Formel I, in welcher $R_3$ Hydroxyniederalkyl bedeutet, können wie oben angegeben zu Verbindungen, in denen $R_3$ Niederalkanoyloxyniederalkyl bedeutet, acyliert werden. Erhaltene Verbindungen der Formel I, in welchen $R_1$ und/oder $R_2$ Wasserstoff bedeutet, können in die entsprechenden (Halogen oder Acyl)-Derivate, z.B. durch Halogenierung, vorzugsweise mit Chlor in Essigsäure oder unter Bedingungen der Friedel-Crafts-Reaktion durch Acylierung mit einem Trihalogenacetyl-halogenid oder einer Halogensulfonsäure, und nachfolgende Behandlung mit einem Alkalimetall-niederalkoxyd, -hydroxyd oder -amid, umgewandelt werden. Erhaltene Carbonsäure- oder Sulfonsäure-Derivate können dann in bekannter Weise, vorzugsweise unter alkalischen Bedingungen hydrolysiert und/oder mit Ammoniak, Mono- oder Diniederalkylaminen amidiert werden. Die erhaltenen Carboxamide können nach konventionellen Methoden zu den entsprechenden Nitrilen dehydratisiert werden. Verbindungen der Formel I, in welchen $R_1$ und/oder $R_2$ Carboxy bedeutet, können beispielsweise erhalten werden, in dem man Verbindungen, worin $R_1$ und/oder $R_2$ Cyan, Carbalkoxy oder Carbamoyl bedeutet, hydrolysiert.

Erhaltene tertiäre Amine, worin $R_3$ sich von Wasserstoff unterscheidet und beispielsweise Niederalkyl oder Arylniederalkyl bedeutet, können in an sich bekannter Weise in die N-Oxide umgewandelt werden. Man arbeitet z.B. mit Wasserstoffsuperoxyd oder organischen Persäuren, z.B. niederen Peralkansäuren oder Perbenzoesäuren, wie Peressigsäure oder m-Chlor-perbenzoesäure, vorzugsweise bei Zimmertemperatur oder mit der letztgenannten Säure, darunter, oder bis 100° mit verdünntem Wasserstoffsuperoxyd, in Gegenwart von Niederalkansäuren, z.B. Essigsäure. Sind lediglich Mono-N-oxide erwünscht, so ist darauf zu achten, dass weitere Oxidationen verhindert werden.

Schliesslich können die Verbindungen der Erfindung in Form von freien Basen oder als Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z.B. durch Behandlung mit einer stärkeren Base, wie mit einem Metallhydroxyd oder Ammoniumhydroxyd, basischen Salz oder einem Kationenaustauscher, z.B. mit einem Alkalimetallhydroxyd- oder -carbonat, umgewandelt werden. Säuren, die therapeutisch verwendbare Säureadditionssalze ergeben, sind z.B. die vorher beschriebenen anorganischen Säuren oder organischen Säuren.

Verbindungen der Formel I, in welchem $R_1$ und/oder $R_2$ Carboxy bedeutet, können auch in die entsprechenden Metall- oder Ammoniumsalze, z.B. durch Behandlung mit Alkali- oder Erdalkalimetallhydroxiden oder -carbonaten, Ammoniak oder mit vorher genannten Aminen, übergeführt werden. Diese oder andere Salze, z.B. die Pikrate, können auch in der Reinigung von den erhaltenen freien Basen verwendet werden. Die Basen werden in ihre Salze übergeführt, die Salze abgetrennt und die Basen aus den Salzen freigesetzt.

Infolge der engen Beziehung zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Erhaltene Isomerengemische von Verbindungen, z.B. solchen der Formel I bis VII, können nach an sich bekannten Methoden, z.B. durch fraktionierte Destillation, Kristallisation und/oder Chromatographie, in die einzelnen Isomeren getrennt werden. Razemische Produkte können in die optischen Antipoden, z.B. bei Trennung ihrer diastereoisomeren Salze, z.B. durch fraktionierte Kristallisation der d- oder ℓ-Tartrate getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen, insbesondere solchen der Formel II, führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen

BAD ORIGINAL

oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer erhalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50% des Aktivstoffes. Einzeldosen für Säuger mit einem Gewicht von ungefähr 50-70 kg können zwischen ungefähr 25-200 mg des aktiven Bestandteils enthalten.

Die nachfolgenden Beispiele a) bis e) sollen beispielsweise im allgemeinen die Herstellung einiger typischer Applikationsformen erläutern, jedoch keineswegs die einzigen Ausführungsformen von solchen darstellen. Besondere Ausführungsformen sind in den Beispielen beschrieben.

a) 100,0 g Wirkstoff werden mit 610,0 g Lactose und 442,0 g Kartoffelstärke vermischt, die Mischung mit einer alkoholischen Lösung von 8 g Gelatine befeuchtet und durch ein Sieb granuliert. Nach dem Trocknen mischt man 60,0 g Talk, 10,0 g Magnesiumstearat und 20,0 g kolloidales

Siliciumdioxid zu und presst die Mischung zu 10'000 Tabletten von je 125 mg Gewicht und 10 mg Wirkstoffgehalt, die gewünschtenfalls mit Teilkerben zur feineren Anpassung der Dosierung versehen sein können.

b) Aus 100,0 g Wirkstoff, 379,0 g Lactose und der alkoholischen Lösung von 6,0 g Gelatine stellt man ein Granulat her, das man nach dem Trocknen mit 10,0 g kolloidalem Siliciumdioxid, 40,0 g Talk, 60,0 g Kartoffelstärke und 5,0 g Magnesiumstearat mischt und zu 10'000 Dragéekernen presst. Diese werden anschliessend mit einem konzentrierten Sirup aus 533,5 g krist. Saccharose, 20,0 g Schellack, 75,0 g arabischem Gummi, 250,0 g Talk, 20,0 g kolloidalem Siliciumdioxid und 1,5 g Farbstoff überzogen und getrocknet. Die erhaltenen Dragées wiegen je 150 mg und enthalten je 10 mg Wirkstoff.

c) 10,0 g Wirkstoff und 1990 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2,0 g gegossen. Sie enthalten je 25 mg Wirkstoff.

d) Zur Bereitung eines Sirups mit 0,25% Wirkstoffgehalt löst man in 3 Litern dest. Wasser 1,5 Liter Glycerin, 42 g p-Hydroxybenzoesäuremethylester, 18 g p-Hydroxybenzoesäure-n-propylester und unter leichtem Erwärmen 25,0 g Wirkstoff, fügt 4 Liter 70%ige Sorbitlösung, 1000 g krist. Saccharose, 350 g Glucose und einen Aromastoff, z.B. 250 g "Orange Peel Soluble Fluid" von Eli Lilly und Co., Indianapolis, oder 5 g natürliches Zitronenaroma und 5 g "Halb und Halb"-Essenz, beide von der Firma Haarmann und Reimer, Holzminden, Deutschland, zu, filtriert die erhaltene Lösung und ergänzt das Filtrat mit dest. Wasser auf 10 Liter.

e) Zur Bereitung einer Tropflösung mit 1,5% Wirkstoffgehalt löst man 150,0 g Wirkstoff und 30 g Natriumcyclamat in einem Gemisch von 4 Liter Aethanol (96%) und 1 Liter Propylenglykol. Andererseits

BAD ORIGINAL

mischt man 3,5 Liter 70%ige Sorbitlösung mit 1 Liter Wasser und fügt die Mischung zur obigen Wirkstofflösung. Hierauf wird ein Aromastoff, z.B. 5 g Hustenbonbon-Aroma oder 30 g Grapefruit-Essenz, beide von der Firma Haarmann und Reimer, Holzminden, Deutschland, zugegeben, das Ganze gut gemischt, filtriert und mit dest. Wasser auf 10 Liter ergänzt.

Die nachfolgenden Beispiele erläutern die Herstellung der neuen Verbindungen der Formel I, sollen jedoch den Umfang der Erfindung in keiner Weise beschränken. Die Temperaturen sind in Celsiusgraden angegeben.

Beispiel 1: Ein Gemisch von 5100 ml Amylalkohol und 918,35 g (9.17 Mol) 1-Methylpiperazin werden in einem 12 Liter fassenden Dreihalskolben, der mit einem Dean-Stark-Aufsatz versehen ist, vorgelegt. Zu diesem Lösungsgemisch werden unter Stickstoff und Rühren 989 ml 10-normaler äthanolischer Chlorwasserstoff rasch hinzugefügt. Das erhaltene Reaktionsgemisch wird zum Rückfluss erhitzt und das Destillat im Dean-Stark-Aufsatz aufgefangen. Sobald die Temperatur des Reaktionsgemisches 131° erreicht, wird der Dean-Stark-Aufsatz entfernt und weitere 918.35 g (917 Mol) 1-Methylpiperazin und 1045,0 g (4.56 Mol) 5-Methylthio-11H-imidazo[1,2-c][1,3]benzodiazepin werden zugegeben. Das Gemisch wird unter Stickstoff weitere 20 Stunden zum Rückfluss erhitzt. Der im Reaktionsgemisch enthaltene Amylalkohol wird im Vakuum bei einer Wasserbadtemperatur von 80° entfernt. Der erhaltene viskos, ölige Rückstand wird in 10 000 ml Dichlormethan gelöst, die Lösung 3-mal mit 4000 ml 4-normaler wässrigen Natriumhydroxidlösung und 6-mal mit 4000 ml Wasser gewaschen. Schliesslich extrahiert man die Dichlormethanlösung mit 3-mal 2000 ml wässriger 6-normaler Salzsäure. Die wässrige Lösung wird danach noch 2-mal mit 2000 ml Dichlormethan ausgewaschen, mit Aktivkohle entfärbt, filtriert und das klare Filtrat mit 1500 ml 29%-igem wässerigem Ammoniak auf einen pH-Wert von 9-10 eingestellt. Das sich abscheidende Oel wird mit 3-mal 4000 ml Dichlormethan extrahiert, die vereinigten Extrakte

werden getrocknet, filtriert und bei 60° Wasserbadtemperatur eingedampft.

Man erhält als Rückstand ein Oel, welches rasch in den festen Zustand übergeht und nach weiterem Trocknen unter Vakuum (5 mmHg/40°) erhält man ein rohes Produkt, welches bei 113-120° schmilzt. Man löst das erhaltene ungereinigte Produkt in 8000 ml heissem (60-70°) Isopropanol, behandelt die Lösung zur Entfärbung mit 200 g Aktivkohle und filtriert diese.

Zu diesem erhaltenen Filtrat fügt man eine Lösung von 760.7 g (8.28 Mol) Maleinsäure in 2500 ml warmem (30°)Isopropanol, wobei das Maleat als Salz ausfällt. Die erhaltene Suspension wird über Nacht bei Zimmertemperatur gerührt, um die Kristallisation zu vervollständigen. Der erhaltene kristalline Niederschlag wird abfiltriert und das erhaltene Produkt 3-mal mit 500 ml kaltem Isopropanol gewaschen und unter Vakuum getrocknet (0,5 mm/50°) und aus Aethanol umkristallisiert. Nach dem Waschen mit Aethanol und Aether erhält man nach dem Trocknen das 5-(4-Methyl-1-piperazinyl)-11H-imidazo-[1,2-c][1,3]benzodiazepin als Monomaleat mit einem Schmelzpunkt von 204-205° (Zersetzung).

Eine Lösung von 2246 g des oben erhaltenen Maleatsalzes in 9000 ml Wasser wird mit 100 g Aktivkohle behandelt und anschliessend filtriert. Die wässrige Lösung wird mit 1000 ml 29%-igem wässerigen Ammoniak auf einen pH-Wert von 9 eingestellt und die dabei erhaltene freie Base wird als Oel getrennt. Das anfallende Oel wird mit 3-mal 2000 ml Dichlormethan extrahiert, die vereinigten Extrakte werden über Natriumsulfat getrocknet, filtriert und bei ungefähr 40° Wasserbadtemperatur eingedampft. Der erhaltene Rückstand wird aus 14 130 ml Heptan umkristallisiert. Der schwach gelbe Rückstand wird gesammelt, mit zweimal je 500 ml Heptan gewaschen und im Vakuum (0,01 mm/50°) getrocknet. Man erhält das 5-(4-Methyl-1-piperazinyl)-11H-imidazo-

[1,2-c][1,3] benzodiazepin als freie Base, die bei einer Temperatur von 123-124° schmilzt.

Der Ausgangsstoff wird wie folgt hergestellt: 24 000 ml absoluter Aethylalkohol und 3240 g (60,0 Mol) Natriummethylat werden in einem 70 Liter Reaktionskolben vorgelegt. Man rührt diese Lösung unter Stickstoff, fügt zu dieser eine weitere Lösung von 8228,4 g (60,0 Mol) o-Nitrotoluol und 8768,4 g (60,0 Mol) Diäthyloxalat auf einmal hinzu. Das entstehende Lösungsgemisch wird 25 Minuten lang zum Rückfluss erhitzt, auf 60° mit Hilfe eines Eisbades heruntergekühlt und anschliessend vorsichtig mit 18 000 ml Wasser versetzt. Danach erwärmt man das Reaktionsgemisch 1 Stunde lang zum Rückfluss, entfernt den grössten Teil des Aethylalkohols, kühlt die Lösung auf 50° und fügt zu dieser eine Lösung von 4140 g (59.6 Mol) Hydroxylamin-Hydrochlorid in 6000 ml Wasser auf einmal hinzu, wobei die Temperatur kontinuierlich auf 50° gehalten wird. Durch Hinzugabe von 6000 ml einer 10 normalen wässrigen Natronlauge wird der pH-Wert des Reaktionsgemisches auf 7 eingestellt. Das Reaktionsgemisch wird über Nacht bei Zimmertemperatur gerührt. Die erhaltene Suspension wird auf 10° gekühlt und der pH-Wert 1 mit 6000 ml wässriger 12-normaler Salzsäure eingestellt, wobei das Rühren des Reaktionsgemisches über Nacht bei einer Temperatur von 10° fortgesetzt wird, um die Freisetzung der freien Säure fortzusetzen. Der Niederschlag wird abgetrennt, 6-mal mit 4000 ml Wasser gewaschen, über Nacht an der Luft getrocknet und anschliessend in 20 000 ml Toluol suspendiert. Die erhaltene Suspension wird 1 Stunde lang unter Stickstoff gerührt, filtriert und der Rückstand 4-mal mit je 2000 ml Toluol und 4 x 2000 ml Petroläther gewaschen und bei 60°/5 mmHg getrocknet. Man erhält das Oxim der 2-Nitrophenylbrenztraubensäure, die bei einer Temperatur von 158-160° (unter Zersetzung) schmilzt.

50 000 ml Wasser, 2940 ml Eisessig und 22,2 Mol des erhaltenen Oxims der 2-Nitrophenylbrenztraubensäure werden in einem 70 Liter Reaktionsgefäss vorgelegt. Die erhaltene Suspension wird erwärmt und

BAD ORIGINAL

unter Stickstoff zwei Stunden lang bei 90° gerührt. Die sich dunkel verfärbte Reaktionslösung, welche als Suspension vorliegt, lässt man langsam abkühlen, rührt diese über Nacht bei Zimmertemperatur, extrahiert diese 5-mal mit je 4000 ml Methylenchlorid, wäscht mit 3-mal je 3000 ml Wasser, trocknet über Magnesiumsulfat und filtriert. Das erhaltene Filtrat wird mit Aktivkohle behandelt, nochmals filtriert und das Lösungsmittel im Vakuum entfernt. Der feste Rückstand wird aus 1000 ml Isopropanol umkristallisiert. Man erhält 2-Nitrophenylacetonitril mit einem Schmelzpunkt von 82-84°.

Man füllt 2250 ml abs. Aethanol und 1500 g (9,25 Mol) 2-Nitrophenylacetonitril in einen 22 Liter Kolben. Die Suspension wird auf 5-10° gekühlt und man leitet in das Gemisch 2,5 Stunden Chlorwasserstoff ein. Das Reaktionsgemisch wird bei 10° unter Stickstoff über Nacht gerührt, dann mit 16000 ml Aether verdünnt und eine Stunde gerührt. Der Niederschlag wird abfiltriert, mit 4 x 1000 ml Aether gewaschen und getrocknet (5 mmHg/40°). Man erhält das Aethyl 2-(2-Nitrophenyl)-acetimidat als Hydrochlorid. F. 122-123° (Zersetzung).

2200 ml Aethanol und 5156 g (8.81 Mol) Aethyl 2-(2-Nitrophenyl)-acetimidate als Hydrochlorid werden in einem 22 Liter Kolben vorgelegt. Die erhaltene Suspension wird unter Stickstoff bei Zimmertemperatur gerührt und mit 1022.9 g (9.73 Mol) Aminoacetaldehyddimethylacetal versetzt. Das Reaktionsgemisch wird weiterhin 1 Stunde lang gerührt und anschliessend mit 1693 ml wässriger 12-normaler Salzsäure auf einmal versetzt, wobei sich die Temperatur des Reaktionsgemisches aufgrund der exothermen Reaktion auf 40° erhöht. Durch weitere Hinzufügung von Wärme wird die Temperatur auf 70-80° erhöht und 30 Minuten lang beibehalten. Das Lösungsgemisch kühlt man mit Hilfe eines Eisbades auf 20° ab, verdünnt mit 2700 ml wässriger 10-normaler Natronlauge, wobei das Reaktionsprodukt ausfällt. Die erhaltene Suspension wird 1 Stunde lang bei 10° unter Stickstoff gerührt, der Niederschlag abfiltriert und 3-mal mit je 2000 ml Wasser gewaschen. Das erhaltene 2-(2-Nitrobenzyl)-imidazol schmilzt bei 155-157°.

BAD ORIGINAL

5672 ml eines 50%-igen wässerigen Aethanols und 2890 g (14.22 Mol) der erhaltenen 2-(2-Nitrobenzyl)-imidazols werden in einem 22 Liter Kolben vorgelegt. Die erhaltene Suspension wird unter Stickstoff gerührt und mit 2400 g (42.97 Mol) Eisenpulver (Korngrösse = 100 mesh) auf einmal versetzt. Das Reaktionsgemisch wird dann auf 70° erwärmt und mit einer Lösung, welche 1,7 ml 12-normale Salzsäure in 8,3 ml absoluten Alkohol enthält, versetzt. Die dabei lebhaft ablaufende exotherme Reaktion erwärmt das Reaktionsgemisch 1,5 Stunden lang zum Rückfluss. Nach Beendigung der exothermen Reaktion fügt man ein Gemisch aus 290 ml einer 12-normalen Salzsäure und 1400 ml absoluten Alkohols innerhalb einer Zeitspanne von 30 Minuten hinzu. Das Reaktionsgemisch wird 2 Stunden zum Rückfluss erwärmt, mit 6500 ml absolutem Aethanol verdünnt und mit 700 ml einer 10-normalen wässrigen Natronlauge auf einen pH-Wert 8-9 eingestellt. Die erhaltene Suspension wird 1 Stunde lang gerührt und anschliessend filtriert. Der erhaltene Rückstand (Filterkuchen) wird mit 1000 ml absolutem Aethanol gewaschen, die Filtrate vereinigt und das Lösungsmittel entfernt. Der aus dem Filtrat erhaltene feste Rückstand wird in 10 000 ml Wasser suspendiert, unter Stickstoff 2 Stunden lang gerührt, abfiltriert und mit 2000 ml Wasser gewaschen und getrocknet. Man erhält das 2-(2-Aminobenzyl)-imidazole mit einem Schmelzpunkt von 153-155°.

42 000 ml Dichlormethan und 5120 g (50.65 Mol) Triäthylamin werden in einem 70 Liter Kolben vorgelegt. Unter Stickstoff und ständigem Rühren werden 4370 g (25.23 Mol) 2-(2-Aminobenzyl)-imidazol hinzugefügt. Die erhaltene Suspension wird auf eine Temperatur von 0-5° abgekühlt und in einer Zeitspanne von über 3 Stunden werden 3 421 g (29.75 Mol) eines 85%igen Thiophosgen enthaltenden Tetrachlorkohlenstofflösung hinzugefügt, wobei die Reaktionstemperatur langsam auf 15° ansteigt. Die Suspension wird weitere 4 Stunden bei 10° und danach über Nacht bei Zimmertemperatur gerührt. Der ausgefallene Niederschlag wird gesammelt, 2-mal mit je 3000 ml Dichlormethan und 5-mal mit je 4000 ml Wasser gewaschen, bei 60° im Vakuum (5 mmHg) getrocknet. Man erhält das 11H-Imidazo[1,2-c][1,3]benzodiazepin-5(6H)-thion von einem Schmelzpunkt von 182-183°.

Zu 20 000 ml absolutem Aethanol werden unter Stickstoff und ständigem Rühren 517.62 g (9.58 Mol) Natriummethylat hinzugefügt. (Ansatz in einem 70 Liter Kolben). Nach halbstündigem Rühren erfolgt vollständige Auflösung, so dass 2063 g (9.58 Mol) 11H-Imidazo[1,2-c][1,3]-benzodiazepin-5(6H)-thion zur weiteren Umsetzung hinzugefügt werden. Nach weiterem 1-stündigem Rühren bei Zimmertemperatur erfolgt wiederum vollständige Auflösung. Zur klaren Lösung werden unter Kühlung auf 1° 1360 g (9.58 Mol) Methylenjodid innerhalb einer Zeitspanne von etwas über 30 Minuten hinzugefügt. Das erhaltene Reaktionsgemisch rührt man 4 Stunden lang bei einer Temperatur von 5° und danach noch über Nacht bei Zimmertemperatur. Die erhaltene trübe Lösung kühlt man auf 5° ab und verdünnt mit 50 000 ml Wasser. Die dabei anfallende Suspension wird danach weitere 4 Stunden lang bei 5° gerührt, der Niederschlag abgetrennt und bei 60° im Vakuum (5 mmHg) getrocknet. Man erhält das 5-Methylthio-11H-imidazo[1,2-c][1,3]benzodiazepin, welches einen Schmelzpunkt von 87-88° aufweist.

In analoger Weise erhält man ausgehend von 4-Chlor-2-nitrophenyl-acetonitril das 8-Chlor-11H-imidazo[1,2-c][1,3]-benzodiazepin-5(6H)-thion vom Schmelzpunkt von 200-201° und das 8-Chlor-5-methylthio-11H-imidazo[1,2-c][1,3]benzodiazepin-hydrochlorid, welches bei einer Temperatur von 255-257° schmilzt.

Die folgenden Ausgangsverbindungen werden auf gleiche Weise aus den entsprechend substituierten 2-Nitrophenyl-acetonitrilen hergestellt:

a) 8-Methyl-5-methylthio-11H-imidazo[1,2-c][1,3]benzodiazepin;
b) 8-Fluor-5-methylthio-11H-imidazo[1,2-c][1,3]benzodiazepin;
c) 8-Methoxy-5-methylthio-11H-imidazo[1,2-c][1,3]benzodiazepin.

Beispiel 2: Zu einer Suspension von 2,46 g 1-[2-([illegible]-Imidazolylmethyl)-phenylcarbamoyl]-4-methylhomopiperazin in 19,4 ml Phosphoroxychlorid werden 1,66 g Phosphorpentachlorid hinzugegeben und das erhaltene Reaktionsgemisch wird weitere [illegible] Stunden lang bei Zimmertemperatur gerührt. Das Gemisch wird zur Trockne eingedampft (im

BAD ORIGINAL

Vakuum), der Rückstand in 45,2 ml Methylenchlorid suspendiert, die erhaltene Suspension wird auf 0° gekühlt und tropfenweise innerhalb einer Zeitspanne von 15 Minuten unter Rühren mit 21,4 ml Triäthylamin versetzt. Das Gemisch lässt man auf Zimmertemperatur erwärmen, rührt weitere 1 1/2 Stunden und giesst das erhaltene Reaktionsgemisch in eine 10%ige wässrige Kaliumcarbonatlösung (Pottasche-lösung). Die dabei sich abscheidende Methylenchloridschicht wird abgetrennt, die wässrige Schicht mit Methylenchlorid gewaschen und die vereinigten Methylenchlorid Extrakte über Magnesiumsulfat getrocknet, mit Aktivkohle entfärbt und zur Trockne eingedampft. Der erhaltene Rückstand wird säulenchromatographisch über 50 g Silicagel gereinigt, wobei ein Lösungsgemisch von Methylenchlorid/Methanol/konz. Ammoniaklösung (300:50:1) als Eluierungsmittel verwendet wird. Man erhält das 5-(4-Methyl-1-homopiperazinyl)-11H-imidazo[1,2-c][1,3]benzodiazepin als Oel. Die erhaltene freie Base wird in Aceton gelöst und mit Maleinsäure behandelt. Man erhält das 5-(4-Methyl-1-homopiperazinyl)-11H-imidazo[1,2-c][1,3]benzodiazepin-monomaleat vom Schmelzpunkt von 160-163°. Das 5-(4-Methyl-1-piperazinyl)-11H-imidazo[1,2-c][1,3]-benzodiazepin-monomaleat, welches im Beispiel 1 beschrieben worden ist, kann in analoger Weise aus dem 1-[2-(2-Imidazolylmethyl)-phenylcarbamoyl]-4-methyl-piperazin erhalten werden.

Der Ausgangsstoff wird wie folgt hergestellt:

Eine Lösung von 32.4 g Chlorameisensäurephenylester in 100 ml Acetonitril wird tropfenweise unter Stickstoff und unter Rühren zu einem Gemisch aus 34,6 g 2-(2-Aminobenzyl)-imidazol und 71 g Triäthylamin in 600 ml Acetonitril bei Zimmertemperatur hinzugegeben. Nach vollendeter Zugabe wird das Reaktionsgemisch 12 Stunden lang zum Rückfluss erhitzt und nach dem Abkühlen auf Zimmertemperatur werden 150 ml Wasser hinzugefügt, eine weitere halbe Stunde wird das Reaktionsgemisch gerührt und auf 5° gekühlt. Der anfallende Niederschlag wird abfiltriert, zweimal mit je 50 ml Wasser und anschliessend 3-mal mit je 33 ml kaltem Aceton gewaschen und getrocknet. Man erhält das 11H-Imidazo[1,2-c][1,3]benzodiazepin-5-

BAD ORIGINAL

(6H)-on, welches bei 255-257° schmilzt.

Wahlweise kann man auch 0,75 g 1,1'-Carbonyldiimidazol zu einer Suspension von 0,79 g 2-(2-Aminobenzyl)-imidazol in 38 ml Methylenchlorid auf einmal hinzugeben und das Gemisch über Nacht bei Zimmertemperatur rühren. Der entstehende Niederschlag wird abgetrennt und aus Methylenchlorid umkristallisiert. Man erhält das ungereinigte 11H-Imidazo[1,2-c][1,3]benzodiazepin-5(6H)-on mit einem Schmelzpunkt von 238-240°.

In analoger Weise kann man unter Verwendung von Phosgen als Zyklisierungsmittel die folgenden Zwischenprodukte erhalten:

a) das 2,3-Dimethyl-11H-imidazo[1,2-c][1,3]benzodiazepin-5(6H)-on;
b) das 8-Chlor-11H-imidazo[1,2-c][1,3]benzodiazepin-5(6H)-on;
c) das 8-Methyl-11H-imidazo[1,2-c][1,3]benzodiazepin-5(6H)-on;
d) das 8-Methoxy-11H-imidazo[1,2-c][1,3]benzodiazepin-5(6H)-on.

Zu einer Suspension von 0,76 g 11H-Imidazo[1,2-c][1,3]benzodiazepin-5(6H)-on in 9 ml Methylenchlorid fügt man 0,41 g N-Methyl-homopiperazin hinzu und rührt das Gemisch weitere 24 Stunden lang bei Zimmertemperatur. Das Reaktionsgemisch wird filtriert und das erhaltene Filtrat im Vakuum zur Trockne eingedampft. Nach dem Umkristallisieren des erhaltenen Rückstandes aus einem Gemisch aus Methylenchlorid/Diäthyläther erhält man das 1-[2-(2-Imidazoyl-methyl)-phenylcarbamoyl]-4-methyl-homopiperazin, welches bei einer Temperatur von 139-143° schmilzt.

In analoger Weise erhält man aus dem 1-Methyl-piperazin das 1-[2-(2-imidazolylmethyl)-phenylcarbamoyl]-4-methylpiperazin mit einem Schmelzpunkt von 172-174°.

Die folgenden Verbindungen werden in analoger Weise, wie oben beschrieben, erhalten:

BAD ORIGINAL

a) 1-[2-(4,5-Dimethyl-2-imidazoylmethyl)-phenylcarbamoyl]-4-methyl-piperazin; und

b) 1-[2-(2-Imidazolylmethyl)-5-chlorphenylcarbamoyl]-4-methyl-piperazin.

Beispiel 3: Eine Lösung von 2,4 g von 5-Thiocyanato-11H-imidazo[1,2-c]-[1,3]benzodiazepin in 5 ml Hexamethylphosphoramid wird auf -5° abgekühlt und tropfenweise unter wirksamen Rühren und unter Stickstoff werden 2,1 g 1-Methyl-piperazin innerhalb einer Zeitspanne von 5 Minuten hinzugefügt. Das Rühren bei -5° wird weitere 10 Minuten lang und danach nach dem Entfernen des Kühlbades noch weitere 10 Minuten fortgesetzt. Das Reaktionsgemisch wird mit 100 ml Aethylacetat verdünnt, zweimal mit Sodalauge gewaschen, über Magnesiumsulfat getrocknet und im Vakuum zur Trockne eingedampft. Zum Rückstand fügt man eine Lösung von 1,2 g Maleinsäure in 3 ml Aceton hinzu und verdünnt mit Diäthyläther, wobei das ungereinigte 5-(4-Methyl-1-piperazinyl)-11H-imidazo[1,2-c][1,3]benzodiazepin-monomaleat kristallin anfällt, Schmelzpunkt 183-186°. Nach dem Umkristallisieren aus Aethanol erhält man wie in Beispiel 1) beschrieben das gereinigte Produkt mit einem Schmelzpunkt von 204-205° (Zersetzung).
Das Ausgangsmaterial wird wie folgt hergestellt:
Eine Suspension von 1,44 g 50%-igem Natriumhydrid in Mineralöl und 100 ml trockenem Tetrahydrofuran wird portionsweise mit 6,45 g 11H-Imidazo[1,2-c][1,3]benzodiazepin-5(6H)-thion versetzt. Die Zugabe erfolgt unter Rühren in einer Stickstoffatmosphäre innerhalb eines Zeitintervalles von 2 Minuten. Das Reaktionsgemisch wird weitere 1 1/2 Stunden bei Zimmertemperatur gerührt. Die erhaltene weisse Suspension wird auf 0° gekühlt und die Lösung tropfenweise mit 3,5 g Bromcyan in 10 ml Tetrahydrofuran versetzt. Das Reaktionsgemisch wird eine halbe Stunde lang bei Zimmertemperatur gerührt und anschliessend im Vakuum bei 45° eingedampft. Der Rückstand wird in Methylenchlorid gelöst, die erhaltene Lösung mit Wasser gewaschen, über Magnesiumsulfat getrocknet, mit Aktivkohle entfärbt und im Vakuum auf ein kleines Volumen eingeengt. Man erhält das 5-Thiocyanato-11H-imidazo[1,2-c][1,3]benzodiazepin, Schmelzpunkt 111-113°,

kristallin beim Verdünnen der Lösung mit Diäthyläther.

Beispiel 4: Zu einer auf 0° gekühlten Lösung von 8,9 g 1-[2-(2-Imidazolylmethyl)-phenylthiocarbamoyl]-4-äthoxycarbonyl-piperazin in 70 ml Acetonitril werden 2,4 g festes Kaliumcarbonat (Pottasche) unter Rühren hinzugefügt, gefolgt von einer tropfenweisen Zugabe einer Lösung von 2,5 g Bromcyan in 10 ml Acetonitril. Man lässt das Reaktionsgemisch über Nacht auf Zimmertemperatur anwärmen, filtriert die festen Bestandteile ab, wäscht diese mit Aethylacetat aus und dampft die vereinigten Filtrate im Vakuum zur Trockne ein. Der Rückstand wird in Methylenchlorid gelöst, die erhaltene Lösung mit Wasser gewaschen, über Magnesiumsulfat getrocknet, mit Aktivkohle entfärbt und im Vakuum eingedampft. Der erhaltene Rückstand wird chromatographisch über 250 g Silicagel gereinigt, wobei man das Lösungsgemisch Aethylacetat/Methanol (9:1) als Eluierungsmittel verwendet. Man erhält das gereinigte 5-(4-Aethoxycarbonyl-1-piperazinyl)-11H-imidazo[1,2-c][1,3]benzodiazepin mit einem Schmelzpunkt von 137-139°.

Die Ausgangsverbindung wird wie folgt hergestellt:

Eine Lösung von 20 g 1-Aethoxycarbonylpiperazin in 400 ml trockenem Tetrahydrofuran wird auf -65° abgekühlt und mit 61,5 ml einer 2,1 molaren Lösung von n-Butyllithium in Hexan tropfenweise über eine Zeitspanne von 15 Minuten versetzt. Das Reaktionsgemisch wird 15 Minuten gerührt und mit einer Lösung von 16,44 ml Chlortrimethylsilan in 68 ml Tetrahydrofuran innerhalb 15 Minuten tropfenweise versetzt. Das Gemisch lässt man über Nacht auf Zimmertemperatur anwärmen und dampft danach dieses im Vakuum zur Trockne ein. Diäthyläther wird zum Rückstand gegeben, man filtriert die festen Bestandteile ab und dampft das Filtrat im Vakuum zur Trockne ein. Der erhaltene Rückstand wird destilliert. Man erhält das 1-Aethoxycarbonyl-4-trimethylsilylpiperazin, K.P. 102-107°/0.1 mmHg.

Zu einer auf -76° gekühlten Lösung von 4,66 ml eines 85%-igen Thiophosgens in 200 ml Diäthyläther fügt man unter Rühren in einer

Stickstoffatmosphäre eine Lösung von 7 g 1-Aethoxycarbonyl-4-trimethylsilylpiperazin in 37 ml Diäthyläther über eine Zeitspanne von 20 Minuten. Man lässt das Reaktionsgemisch über Nacht auf Zimmertemperatur aufwärmen. Die Suspension wird filtriert und das Filtrat zur Trockne eingedampft. Der Rückstand wird aus einem Lösungsmittelgemisch von Methylenchlorid/Hexan umkristallisiert. Man erhält das 4-Aethoxycarbonyl-1-piperazinyl-thiocarbonyl-chlorid mit einem Schmelzpunkt von 107-110°.

Zu einer Suspension von 3,8 g 2-(2-Aminobenzyl)-imidazol in 38 ml Tetrahydrofuran und 3,23 ml Triäthylamin wird tropfenweise eine Lösung von 5,5 g 4-Aethoxycarbonyl-1-piperazinyl-thiocarbonyl-chlorid in 10 ml Methylenchlorid bei Zimmertemperatur hinzugefügt. Das Reaktionsgemisch wird eine Woche gerührt und die dabei anfallende Suspension filtriert. Das Filtrat wird zuerst mit einer 10%-igen wässrigen Kaliumcarbonatlösung (Pottasche) gewaschen, danach mit Wasser gewaschen, getrocknet und anschliessend zur Trockne eingedampft. Man erhält das amorphe 1-[2-(2-Imidazolyl-methyl)-phenylthiocarbamoyl]-4-äthoxycarbonylpiperazin, welches an Hand von NMR-Spektren charakterisiert wird.

Beispiel 5: Gemäss den in den vorhergehenden Beispielen illustrierten Methoden werden auch die folgenden Verbindungen der Formel I, insbesondere der Formel II, ausgehend von äquivalenten Mengen entsprechender Ausgangsstoffe, erhalten. $R_1$ und $R_2$ bedeuten Wasserstoff und die Gruppe $C_nH_{2n}$ bedeutet Aethylen.

| Nr. | $R_3$ | $R_4$ | Salz | F.p. |
|---|---|---|---|---|
| 1 | $CH_2CH_2OH$ | H | - | 143-144° |
| 2 | $CH_2CH_2OH$ | Cl | HCl | 225° (Zers.) |
| 3 | $CH_3$ | Cl | 2HCl | 226-228° (Zers.) |
| 4 | $CH_3$ | H | 2HCl | >250° (Zers.) |
| 5 | $CH_3$ | H | HCl | 217-220° |

BAD ORIGINAL

Beispiel 6: Ein Gemisch von 315 mg 1-[2-(2-Imidazolylmethyl)-phenylthiocarbamoyl]-4-methylpiperazin, 3,3 ml Dimethylformamid, 276 mg Kaliumcarbonat (Pottasche), 116 mg Bromcyan und 50 mg 8-Kronen-6-äther wird unter Stickstoff bei Zimmertemperatur 3 Stunden gerührt. Das Gemisch wird mit Aethylacetat verdünnt, mit gesättigter wässriger Natriumchloridlösung gewaschen, getrocknet und eingedampft. Der Rückstand wird in Aceton gelöst, die Lösung mit 116 mg Maleinsäure behandelt und mit Diäthyläther verdünnt. Man erhält das 5-(4-Methyl-1-piperazinyl)-11H-imidazo[1,2-c][1,3]benzodiazepin-monomaleat. Das Produkt ist mit demjenigen des in Beispiel 1 erhaltenen identisch.

Der Ausgangsstoff wird wie folgt hergestellt:

Ein Gemisch von 2,1 g 11H-Imidazo[1,2-c][1,3]benzodiazepin-5(6H)-thion, 23 ml Methylenchlorid und 1,0 g 1-Methylpiperazin wird bei Zimmertemperatur 15 Stunden gerührt. Das erhaltene kristalline Produkt wird abfiltriert und mit Methylenchlorid gewaschen. Man erhält das 1-[2-(2-Imidazolylmethyl)-phenylthiocarbamoyl]-4-methyl-piperazin.

Beispiel 7: Ein Gemisch von 9,5 g 5-Methylthio-11H-imidazo[1,2-c]-[1,3]benzodiazepin-hydrochlorid, 3,62 g Piperazin und 350 ml Amylalkohol wird unter Rühren in einer Stickstoffatmosphäre 20 Stunden zum Rückfluss erhitzt. Das Lösungsmittel wird unter Vakuum entfernt, der erhaltene Rückstand mit Methylenchlorid tituriert, mit 2-normaler wässriger Natriumhydroxydlösung gewaschen, über Magnesiumsulfat getrocknet und zur Trockne eingedampft. Der Rückstand wird in 10 ml Methanol gelöst und mit 2-normaler ätherischer Chlorwasserstoffsäure behandelt. Man erhält das 5-(4H-1-Piperazinyl)-11H-imidazo[1,2-c]-[1,3]benzodiazepin-dihydrochlorid.

Beispiel 8: Zu einer Lösung von 0,2 g 5-(4-Carboäthoxy-1-piperazinyl]-11H-imidazo[1,2-c][1,3]benzodiazepin in 2 ml trockenem Tetrahydrofuran werden 100 mg Lithiumaluminiumhydrid auf einmal hinzugegeben und unter Stickstoff das Reaktionsgemisch zum Rückfluss erhitzt. Man lässt das Gemisch auf Zimmertemperatur abkühlen, rührt diese nach Zugabe von 0,2 ml einer 30%-igen wässrigen Natronlauge und filtriert. Das Filtrat wird zur Trockne eingedampft und das erhaltene Produkt gereinigt. Man erhält das 5-(4-Methyl-1-piperazinyl]-11H-imidazo[1,2-c][1,3]benzodiazepin, welches mit dem im Beispiel 1 erhaltenen identisch ist. F.p. 123-124°.

Beispiel 9: Zu einer Lösung von 82 mg von 5-(4-Methyl-1-piperazinyl)-11H-imidazo[1,2-c][1,3]benzodiazepin in 1 ml Methylenchlorid werden 74 mg m-Chlorperbenzoesäure bei 0° zugefügt. Das Gemisch wird bei 0° über Nacht gerührt, danach mit 1 ml Diäthyläther verdünnt, ein Aequivalent einer ätherischen Chlorwasserstoffsäurelösung (Salzsäurelösung) hinzugefügt und der erhaltene Niederschlag abfiltriert. Nach dem Umkristallisieren erhält man 5-(4-Methyl-4-oxido-1-piperazinyl)-11H-imidazo[1,2-c][1,3]benzodiazepin-hydrochlorid.

Beispiel 10: Zu einer Lösung von 100 ml 5-(4-Benzyloxycarbonyl-1-piperazinyl)-11H-imidazo[1,2-c][1,3]benzodiazepin in 0,3 ml Essigsäure werden 0,35 ml einer 2-normalen Lösung von Bromwasserstoff in Essigsäure hinzugegeben. Das Gemisch wird eine Stunde lang auf 100° erhitzt und danach über Nacht bei Zimmertemperatur gerührt. Man fügt Diäthyläther hinzu, wobei das 5-(4H-1-Piperazinyl)-11H-imidazo[1,2-c]-[1,3]benzodiazepin-hydrobromid ausfällt.

Der Ausgangsstoff wird in analoger Weise wie unter Beispiel 4 unter Austausch von 1-Aethoxycarbonylpiperazin durch die äquivalente Menge von 1-Benzyloxycarbonylpiperazin erhalten.

Beispiel 11: Ein Gemisch von 265 mg 5-(4H-1-Piperazinyl)-11H-imidazo-[1,2-c][1,3]benzodiazepin, 0,5 g Kaliumcarbonat, 0,142 g Methyljodid und 2 ml Aceton wird über Nacht bei Zimmertemperatur gerührt und danach eingedampft. Man setzt Wasser zum Rückstand zu und extrahiert das Gemisch mit Methylenchlorid. Die vereinigten Extrakte werden über Magnesiumsulfat getrocknet, eingedampft und man erhält nach dem Reinigen das 5-(4-Methyl-1-piperazinyl)-11H-imidazo[1,2-c][1,3]benzodiazepin.

Beispiel 12: Gemäss den in den vorhergehenden Beispielen illustrierten Methoden werden auch die folgenden Verbindungen der Formel I, ausgehend von äquivalenten Mengen entsprechender Ausgangsstoffe, erhalten. Die Substituenten $R_5$ bis $R_7$ bedeuten Wasserstoff:

| Nr. | $R_1$ | $R_2$ | $R_3$ | $R_4$ | $C_nH_{2n}$ |
|---|---|---|---|---|---|
| 1 | $CH_3$ | H | $CH_3$ | H | $(CH_2)_2$ |
| 2 | $CH_3$ | $CH_3$ | $CH_3$ | H | $(CH_2)_2$ |
| 3 | H | H | $CH_3$ | 8-$CF_3$ | $(CH_2)_2$ |
| 4 | H | H | $CH_3$ | 8-F | $(CH_2)_2$ |
| 5 | H | H | $CH_3$ | 8-$OCH_3$ | $(CH_2)_2$ |
| 6 | H | H | $CH_3$ | 8-OH | $(CH_2)_2$ |
| 7 | H | H | $CH_3OCH_2CH_2$ | H | $(CH_2)_2$ |
| 8 | H | H | $CH_3COOCH_2CH_2$ | H | $(CH_2)_2$ |
| 9 | H | H | $CH_3$ | 8-$CH_3$ | $(CH_2)_2$ |

Beispiel 13: Herstellung von 10 000 Tabletten mit einem Gehalt von je 25 mg der aktiven Substanz:

Bestandteile:

| | |
|---|---|
| 5-(4-Methyl-1-piperazinyl)-11H-imidazo[1,2-c][1,3]benzodiazepin | 250.00 g |
| Milchzucker | 957.00 g |
| Maisstärke | 75.00 g |

| | |
|---|---|
| Polyäthylenglykol 6000 | 75.00 g |
| Talkpulver | 75.00 g |
| Magnesiumstearat | 18.00 g |
| gereinigtes Wasser | q.s. |

Verfahren:

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 6,4 mm Durchmesser, welche eine Bruchrille aufweisen, gepresst.

Beispiel 14: Herstellung von 10 000 Kapseln mit einem Gehalt von je 50 mg der aktiven Substanz:

Bestandteile:

| | |
|---|---|
| 5-(4-Methyl-1-piperazinyl)-11-imidazo[1,2-c][1,3]-benzodiazepin-monomaleat | 500.00 g |
| Milchzucker | 1400.00 g |
| Talkpulver | 100.00 g |

Verfahren: Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 34 werden mit je 200 mg des Gemisches mit einer Füllmaschine gefüllt.

BAD ORIGINAL

In analoger Weise oder wie weiter vorne beschrieben werden Tabletten oder Kapseln von anderen erfindungsgemässen Verbindungen, z.B. solchen, die in den weiteren Beispielen hier illustriert sind, hergestellt.

Beispiel 15: Ein Gemisch von 10 g 1-[2-(4-Methyl-2-imidazolylmethyl)-phenylcarbamoyl]-4-methylpiperazin,. 86 ml Phosphoroxychlorid und 7.24 g Phosphorpentachlorid wird bei Zimmertemperatur 4 Stunden lang gerührt und anschliessend zur Trockne eingedampft. Der Rückstand wird in 186 ml Methylenchlorid suspendiert, die Suspension auf 0° gekühlt und 55,2 ml Triäthylamin werden tropfenweise innerhalb einer Zeitspanne von 15 Minuten zugefügt. Man rührt das erhaltene Gemisch über Nacht bei Zimmertemperatur, giesst dieses in kaltes Wasser, welches mit 10%-iger wässriger Natronlauge basisch gestellt worden ist und extrahiert mit Methylenchlorid. Die Methylenchloridextrakte werden nochmals mit 2-normaler Salzsäure extrahiert. Die sauren Extrakte werden mit 2-normaler wässriger Natronlauge basisch gestellt und 3-mal mit Methylenchlorid extrahiert. Die organischen Extrakte werden über Magnesiumsulfat getrocknet, mit Aktivkohle entfärbt und zur Trockne eingedampft. Der Rückstand wird chromatographisch über 180 g Silicagel unter Verwendung von einem Lösungsgemisch von Methylenchlorid/Methanol/wässrige Ammoniaklösung/(300:50:1) als Eluierungsmittel gereinigt. Man erhält ein schaumähnlich gelockertes Produkt, welches in Aceton gelöst wird und mit der äquivalenten Menge Maleinsäure versetzt wird. Beim Verdünnen mit Diäthyläther erhält man das 2-Methyl-5-(4-methyl-1-piperazinyl)-11H-imidazo[1,2-c][1,3]-benzodiazepin-monomaleat mit einem Schmelzpunkt von 195-197°.

Der Ausgangsstoff wird wie folgt hergestellt:

Eine Lösung von äthanolischer Natriumalkoholatlösung, hergestellt durch lösen von 4,48 g metallischem Natrium in 112 ml absolutem Aethanol, wird tropfenweise zu einer Suspension von 47.84 g Aethyl 2-(2-Nitrophenyl)-acetamidat-hydrochlorid in 224 ml Aethanol hinzugegeben und das Gemisch wird bei Zimmertemperatur 1 Stunde lang

gerührt. Das sich bildende Natriumchlorid wird abfiltriert und 22,82 g des Aethylenketals des 1-Amino-2-propanons werden zum Filtrat hinzugefügt und das Gemisch wird über Nacht bei Zimmertemperatur gerührt. Der unlösliche Niederschlag wird abgetrennt und das Filtrat im Vakuum zur Trockne eingedampft. Der erhaltene Rückstand wir in 470 ml konz. Salzsäure gelöst und die Lösung wird eine Stunde lang zum Rückfluss erhitzt. Das Gemisch wird einmal mit Diäthyläther gewaschen, mit 2-normaler wässriger Natronlauge basisch gestellt und anschliessend 3-mal mit Aethylacetat extrahiert. Die Extrakte werden über Magnesiumsulfat getrocknet, mit Aktivkohle entfärbt und eingedampft. Der erhaltene Rückstand wird aus einem Gemisch aus Methylenchlorid/Aether umkristallisiert. Man erhält das 4-Methyl-2-(2-nitrobenzyl)-imidazol mit einem Schmelzpunkt von 125-128°.

Ein Gemisch von 23,44 g 4-Methyl-2-(2-nitrobenzyl)-imidazol, 2,34 g eines 10%-igen Palladium-auf-Kohle-Katalysators und 234 ml Aethanol wird 4 Stunden lang bei Zimmertemperatur und 3 Atmosphären (42 psi) hydriert. Der Katalysator wird abfiltriert und das erhaltene Filtrat zur Trockne eingedampft. Man erhält das 4-Methyl-2-(2-aminobenzyl)-imidazol, welches im NMR-Spektrum folgende Signale: $\delta$ 2.09, 3.78 und 6.08 aufweist.

Ein Gemisch aus 18,61 g 4-Methyl-2-(2-aminobenzyl)-imidazol, 16,12 g 1,1'-Carbonyldiimidazol und 375 ml Methylenchlorid wird bei Zimmertemperatur über Nacht gerührt. Das Methylenchlorid des Reaktionsgemisches wird auf ein kleines Volumen eingeengt und das verbliebene Gemisch auf 0° gekühlt. Die ausgefallenen festen Bestandteile werden abfiltriert und mit Diäthyläther gewaschen. Man erhält das 2-Methyl-11H-imidazo[1,2-c][1,3]benzodiazepin-5(6H)-on mit einem Schmelzpunkt von 234.5-236.5°.

Ein Gemisch von 16 g 2-Methyl-11H-imidazo[1,2-c][1,3]benzodiazepin-5(6H)-on, 9,68 g N-Methylpiperazin und 160 ml Methylenchlorid wird über Nacht bei Zimmertemperatur gerührt. Das Reaktionsgemisch wird über Aktivkohle entfärbt und im Vakuum eingedampft. Der Rückstand

wird aus Methanol/Diäthyläther umkristallisiert, wobei man das 1-[2-(4-Methyl-2-imidazolylmethyl)-phenylcarbamoyl]-4-methylpiperazin mit einem Schmelzpunkt von 177-179° erhält.

Beispiel 16: Zu einer Lösung von 5-(4-Methyl-1-piperazinyl)-11H-imidazo[1,2-c][1,3]benzodiazepin in 50 ml Methylenchlorid werden in Portionen aufgeteilt 3.75 g m-Chlorperbenzoesäure unter Rühren bei einer Temperatur von 0° hinzugefügt. Das Gemisch wird über Nacht bei Zimmertemperatur gerührt und zur Trockne eingedampft. Der erhaltene schaumähnliche Rückstand wird durch einen Ionenaustauscher, der 100 g Amberlit-IRA-400 Ionenaustauscherharz enthält, geschickt, wobei man Wasser als Eluierungsmittel verwendet. Nach dem Abdampfen des Eluierungsmittels (Wasser) erhält man das 5-(4-Methyl-4-oxido-1-piperazinyl)-11H-imidazo[1,2-c][1,3]benzodiazepin von schaumähnlicher Konsistenz, die einen Rf-Wert von 0,173 auf Silicagel beschichteten Platten aufweist, wobei man als Eluierungsmittel ein Gemisch aus Methylenchlorid/Methanol/wässrigen Ammoniak (150:50:1) verwendet. Das erhaltene Produkt ist mit dem im Beispiel 9 erhaltenen Produkt identisch.

Beispiel 17: Ein Gemisch aus 8,67 g 5-Methylthio-11H-imidazo[1,2-c]-[1,3]benzodiazepin, 3,38 g Piperazin und 326 ml Amylalkohol wird unter Stickstoff 6 Tage lang zum Rückfluss erhitzt und danach im Vakuum zur Trockne eingedampft. Der Rückstand wird in Methylenchlorid gelöst und die Lösung successiv mit einer 10%-igen wässrigen Kaliumcarbonatlösung und Sodalauge gewaschen, über Magnesiumsulfat getrocknet, mit Hilfe von Aktivkohle entfärbt und zur Trockne eingedampft. Der Rückstand wird chromatographisch über 300 g Silicagel unter Verwendung von Methylenchlorid/Methanol/wässrigem Ammoniak (150:50:1) als Eluierungsmittel gereinigt, wobei das 5-(4H-1-Piperazinyl)-11H-imidazo[1,2-c][1,3]benzodiazepin als Oel anfällt. Das erhaltene Oel wird mit 2,74 g Maleinsäure in Aceton behandelt und man erhält das 5-(4H-1-Piperazinyl)-11H-imidazo[1,2-c][1,3]-benzodiazepin-bismaleat, welches bei 171.5-173.5° schmilzt.

Beispiel 18: Ein Gemisch aus 0,2 g 5-(4-Aethoxycarbonyl-1-piperazinyl)-11H-imidazo[1,2-c][1,3]benzodiazepin, 10 ml Tetrahydrofuran und 50 mg Lithiumaluminiumhydrid wird über Nacht unter Rühren zum Rückfluss erhitzt. Das erhaltene Reaktionsgemisch wird auf 0° gekühlt und der Ueberschuss des Lithiumaluminiumhydrids wird durch Zugabe von Aethylacetat vernichtet. Danach wird das Reaktionsgemisch in kaltes Wasser gegossen und mit Aethylacetat extrahiert. Die vereinigten Extrakte werden getrocknet und zur Trockne eingedampft, wobei man nach der Reinigung (s. Beispiel 1) 5-(4-Methyl-1-piperazinyl)-11H-imidazo-[1,2-c][1,3]benzodiazepin erhält, welches mit dem erhaltenen Produkt in Beispiel 1 identisch ist.

Beispiel 19: Ein Gemisch aus 0,1 g 5-(4H-Piperazinyl)-11H-imidazo-[1,2-c][1,3]benzodiazepin, 0,058 g Methyljodid, 0,16 g Kaliumcarbonat und 1 ml Dimethylformamid wird über Nacht bei Zimmertemperatur gerührt. Das Reaktionsgemisch wird in kaltes Wasser gegossen und danach das Lösungsgemisch 3-mal mit Aethylacetat extrahiert. Die vereinigten Extrakte werden mit Sodalauge gewaschen, getrocknet und im Vakuum eingedampft, wobei man nach dem Reinigen (s. Beispiel 1) das 5-(4-Methyl-1-piperazinyl)-11H-imidazo[1,2-c][1,3]benzodiazepin erhält, welches mit dem erhaltenen Produkt in Beispiel 1 identisch ist.

Vermeidungs-Reaktionen der Ratte

Im vorne beschriebenen Testverfahren ergibt eine Auswahl der neuen Verbindungen die folgenden Werte

| Verbindung des Beispiels | Dosis mg/kg p.o. | Anzahl Tiere | Anzahl der Fehlverhalten (im Vergleich zum Trägermaterial) | | |
|---|---|---|---|---|---|
| | | | 30 Minuten | 90 Minuten | 210 Minuten |
| 1 | 10 | 6 | +31 | +13 | +14 |
| 1 | 30 | 6 | [illegible] | +49 | +40 |
| 5/2 | 30 | 3 | +22 | +19 | + 8 |
| 5/3 | 10 | 3 | +10 | +14 | +15 |

Die Abnahme der Vermeidungs-Reaktionen zeigt die neuroleptische Wirkung, die sich in der Zunahme der Anzahl der Fehlverhalten äussert.

Patentansprüche: (für alle benannten Länder ausser Oesterreich)

1. Verbindungen der allgemeinen Formel I

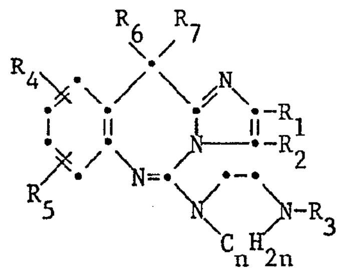

(I),

worin jedes der Symbole $R_1$ und $R_2$ Wasserstoff, Niederalkyl, Niederalkanoyl, Halogen, Cyan, Carboxy, Niederalkoxycarbonyl, Carbamoyl, Sulfamoyl, Mono- oder Di-niederalkyl-(carbamoyl oder -sulfamoyl) bedeutet, $C_nH_{2n}$ für Niederalkylen steht, welches die zwei Stickstoffatome durch 2 oder 3 Kohlenstoffatome trennt, und $R_3$ für Wasserstoff, Niederalkyl, Niederalkenyl, Niederalkynyl, Niederalkanoyl, Arylniederalkyl, Niederalkoxycarbonyl, Phenylniederalkoxycarbonyl, oder (Hydroxy, Niederalkanoyloxy, Aryloxy oder Niederalkoxy)-niederalkyl steht, worin der Niederalkylrest mindestens 2 Kohlenstoffatome enthält, $R_4$ und $R_5$ unabhängig voneinander Wasserstoff, Niederalkyl, Niederalkoxy, Niederalkylthio, Halogen, Trifluormethyl, Hydroxy, Niederalkanoyloxy, Sulfamoyl, Mono- oder Diniederalkylsulfamoyl, und $R_6$ und $R_7$ Wasserstoff oder Niederalkyl bedeuten; ihre N-Oxide, ihre Niederalkyl quaternären Derivate, und Salze.

2. Verbindungen der im Anspruch 1 gezeigten Formel I, worin jedes der Symbole $R_1$ und $R_2$ Wasserstoff, Niederalkyl, Cyan, Carboxy, Niederalkoxycarbonyl oder Carbamoyl, und n die ganze Zahl 2-4 bedeuten, $R_3$ für Wasserstoff, Niederalkyl, Niederalkoxycarbonyl oder Hydroxy-niederalkyl mit 2-4 Kohlenstoffatomen im Niederalkylrest steht, $R_4$ Wasserstoff, Niederalkyl, Niederalkoxy, Niederalkylthio, Halogen- oder Trifluormethyl bedeutet, und $R_5$ für Wasserstoff steht, und $R_6$ und $R_7$ die Bedeutung von Wasserstoff oder Niederalkyl haben, ihre N-Oxide, Niederalkyl quaternären Derivate, und Salze.

3. Verbindungen der im Anspruch 1 gezeigten Formel I, worin jedes der Symbole $R_1$ und $R_2$ Wasserstoff, Methyl, Aethyl, Cyano, Carboxy, Niederalkoxycarbonyl mit 1-3 Kohlenstoffatomen im Niederalkoxyrest oder Carbamoyl bedeutet, n eine ganze Zahl 2 oder 3 bedeutet, $R_3$ für Wasserstoff, Niederalkyl mit 1-3 Kohlenstoffatomen, Niederalkoxycarbonyl mit 1-3 Kohlenstoffatomen im Niederalkoxyrest, Hydroxyäthyl oder Hydroxypropyl steht, $R_4$ Wasserstoff, Methyl, Methoxy, Methylthio, Chlor oder Trifluormethyl, $R_5$ Wasserstoff und $R_6$ und $R_7$ Wasserstoff oder Methyl bedeuten, ihre N-Oxide, Methyl quaternären Derivate, und Salze.

4. Verbindungen der Formel II

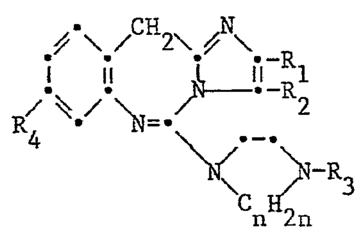

(II),

worin jedes der Symbole $R_1$ und $R_2$ unabhängig voneinander Wasserstoff oder Niederalkyl bedeutet, $R_3$ für Wasserstoff, Niederalkyl oder Hydroxy-niederalkyl steht, worin Hydroxy vom Stickstoffatom durch mindestens 2 Kohlenstoffatome getrennt ist, $R_4$ Wasserstoff, Niederalkyl, Niederalkoxy, Niederalkylthio, Halogen oder Trifluormethyl bedeutet, $C_nH_{2n}$ für Aethylen oder Propylen steht, ihre N-Oxide und Salze.

5. Verbindungen der im Anspruch 4 gezeigten Formel II, worin jedes der Symbole $R_1$ und $R_2$ unabhängig voneinander Wasserstoff oder Methyl, $R_3$ Wasserstoff, Methyl, Aethyl, Propyl, 2-Hydroxyäthyl oder 3-Hydroxypropyl bedeuten, und $R_4$ für Wasserstoff, Methyl, Methoxy, Fluor, Chlor oder Trifluormethyl steht, $C_nH_{2n}$ Aethylen oder Propylen bedeutet, ihre N-Oxide und therapeutisch verwendbaren Salze.

BAD ORIGINAL

6. Verbindungen der im Anspruch 4 gezeigten Formel II, worin $R_1$ und $R_2$ unabhängig voneinander Wasserstoff oder Methyl, und $R_3$ Wasserstoff, Methyl, Aethyl, Propyl, 2-Hydroxyäthyl bedeuten, und $R_4$ für Wasserstoff, Methyl, Fluor, Chlor oder Trifluormethyl steht und $C_nH_{2n}$ Aethylen bedeutet, und ihre therapeutisch verwendbaren Salze.

7. 5-(4-Methyl-1-piperazinyl)-11H-imidazo[1,2-c][1,3]benzodiazepin und seine therapeutisch verwendbaren Salze.

8. 5-[4-(2-Hydroxyäthyl)-piperazinyl]-11H-imidazo[1,2-c][1,3]benzodiazepin und seine therapeutisch verwendbaren Salze.

9. 5-(4-Methyl-1-homopiperazinyl)-11H-imidazo[1,2-c][1,3]benzodiazepin und seine therapeutisch verwendbaren Salze.

10. 5-(4-Aethoxycarbonyl-1-piperazinyl)-11H-imidazo[1,2-c][1,3]benzodiazepin und seine therapeutisch verwendbaren Salze.

11. 2-Methyl-5-(4-methyl-1-piperazinyl)-11H-imidazo[1,2-c][1,3]benzodiazepin und seine therapeutisch verwendbaren Salze.

12. 5-(4-Methyl-4-oxido-1-piperazinyl)-11H-imidazo[1,2-c][1,3]benzodiazepin und seine therapeutisch verwendbaren Salze.

13. 5-(4H-1-Piperazinyl)-11H-imidazo[1,2-c][1,3]benzodiazepin und seine therapeutisch verwendbaren Salze.

14. Pharmazeutische Präparate enthaltend Verbindungen der im Anspruch 1 gezeigten Formel I, ihre N-Oxide, ihre Niederalkyl quaternären Derivate, und Salze, zusammen mit einem pharmazeutischen Trägermaterial.

15. Die im Anspruch 1 genannten Verbindungen zur Anwendung in einem Verfahren zur therapeutischen Behandlung des menschlichen oder tierischen Körpers.

16. Die im Anspruch 1 genannten Verbindungen als neuroleptische Mittel.

17. Die im Anspruch 1 genannten Verbindungen als antihistaminische Mittel.

18. Verwendung der im Anspruch 1 genannten Verbindungen zur Herstellung von pharmazeutischen Präparaten.

19. Verfahren zur Herstellung von den im Anspruch 1 genannten Verbindungen der Formel I, dadurch gekennzeichnet, dass man
a) eine Verbindung der Formel III

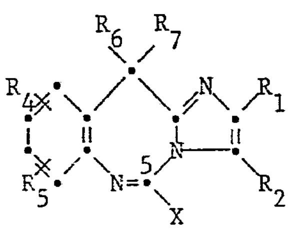

(III),

worin X eine zusammen mit Wasserstoff oder einem Alkalimetall abspaltbare Gruppe bedeutet und die anderen Symbole die unter der Formel I angegebenen Bedeutungen haben, mit einer Verbindung der Formel IV

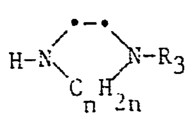

(IV)

oder einem Alkalimetallderivat davon, in welcher $R_3$ die unter der Formel I angegebene Bedeutung hat, kondensiert, oder

b) eine Verbindung der Formel VI

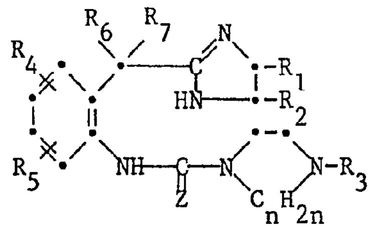

(VI)

unter entwässernden, dehydrosulfurierenden und desaminierenden Bedingungen ringschliesst, worin Z ein Sauerstoff- oder Schwefelatom oder NH bedeutet, und die anderen Symbole die oben angegebenen Bedeutungen haben, und, wenn eine Verbindung der Formel I erwünscht ist, worin $R_3$ Niederalkyl bedeutet, in eine Verbindung der Formel I, worin $R_3$ Wasserstoff oder ein Alkalimetallatom ist, einen genannten Rest einführt, und/oder, wenn eine Verbindung der Formel I erwünscht ist, worin $R_3$ Niederalkyl bedeutet, eine Verbindung, in der anstelle von Niederalkyl ein Niederalkenyl- oder Niederalkynyl steht, diesen zu Niederalkyl reduziert, und/oder, wenn eine Verbindung erwünscht ist, worin $R_3$ Hydroxy-niederalkyl bedeutet, eine Verbindung, worin $R_3$ Wasserstoff oder ein Alkalimetallatom bedeutet, mit entsprechenden Oxiranen oder mit reaktionsfähigen Estern von mono-veresterten Niederalkandiolen umsetzt, und/oder wenn eine Verbindung erwünscht ist, worin $R_3$ Hydroxy-niederalkyl bedeutet, eine erhaltene Verbindung, worin $R_3$ für Wasserstoff steht, zuerst mit reaktionsfähigen Derivaten von entsprechenden Glykolen, Glykolsäuren oder Dicarbonsäuren umsetzt und die erhaltenen Verbindungen zu solchen, in welchen $R_3$ Hydroxyniederalkyl bedeutet hydrolysiert oder reduziert, und/oder, wenn eine Verbindung erwünscht ist, worin $R_3$ Niederalkoxycarbonyl bedeutet, in einem Endprodukt, worin $R_3$ Niederalkyl bedeutet, dieses in eine Niederalkoxycarbonylgruppe überführt, und/oder, wenn eine Verbindung erwünscht ist, worin $R_3$ einen im Anspruch 1 definierten Acylrest bedeutet, eine erhaltene Verbindung, worin $R_3$ für Wasserstoff steht, acyliert, und/oder, wenn eine Verbindung

erwünscht ist, worin $R_3$ Wasserstoff bedeutet, eine erhaltene Verbindung, worin $R_3$ einen Acylrest bedeutet, hydrolysiert, und/oder, wenn eine Verbindung erwünscht ist, worin $R_3$ Methyl bedeutet, in einem erhaltenen Produkt, worin $R_3$ Niederalkoxycarbonyl oder Phenylniederalkoxycarbonyl bedeutet, diese Reste zu Methyl reduziert, und/oder, wenn eine Verbindung erwünscht ist, worin $R_1$ und/oder $R_2$ Halogen bedeutet, ein erhaltenes Produkt, worin $R_1$ und/oder $R_2$ Wasserstoff bedeutet, halogeniert, und/oder, wenn eine Verbindung erwünscht ist, worin $R_1$ und/oder $R_2$ Carboxy, Carboniederalkoxy oder ein im Anspruch 1 genannter Carbamoylrest ist, eine erhaltene Verbindung, worin $R_1$ und/oder $R_2$ Wasserstoff bedeutet, mit einem Trihalogenacetylhalogenid umsetzt und nachfolgend mit einem Alkalimetall-niederalkoxyd, -hydroxyd oder -amid behandelt, und/oder, wenn eine Verbindung erwünscht ist, worin $R_1$ und/oder $R_2$ Sulfamoyl, Mono- oder Di-niederalkylsulfamoyl bedeutet, ein Produkt, worin $R_1$ und/oder $R_2$ Wasserstoff bedeutet, mit einer Halogensulfonsäure umsetzt und nachfolgend mit Ammoniak, einem Mono- oder Di-niederalkylamin behandelt, und/oder, wenn eine Verbindung erwünscht ist, worin $R_1$ und/oder $R_2$ Cyan bedeutet, ein Produkt, worin $R_1$ und/oder $R_2$ für Carbamoyl steht, dehydratisiert, und/oder, wenn eine Verbindung erwünscht ist, worin $R_1$ und/oder $R_2$ Carboxy ist, ein Produkt, worin $R_1$ und/oder $R_2$ Cyan, Carbalkoxy oder Carbamoyl ist, hydrolysiert, und/oder, wenn ein N-Oxid erwünscht ist, ein Produkt, worin $R_3$ sich von Wasserstoff unterscheidet, oxidiert, und/oder, wenn ein quaternäres Niederalkylderivat erwünscht ist, ein Produkt, worin $R_3$ sich von Wasserstoff unterscheidet oder $R_3$ Wasserstoff bedeutet, mit einem reaktionsfähig veresterten Niederalkanol umsetzt, und/oder, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen

Isomeren oder Razemate auftrennt, und/oder, wenn erwünscht, erhaltene Razemate in die optischen Antipoden aufspaltet.

20. Die nach dem Verfahren des Anspruchs 19 erhältlichen Verbindungen.

Patentansprüche

(Oesterreich)

1. Verfahren zur Herstellung von Verbindungen der Formel I

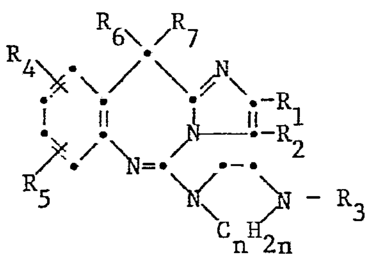

(I),

worin jedes der Symbole $R_1$ und $R_2$ Wasserstoff, Niederalkyl, Niederalkanoyl, Halogen, Cyan, Carboxy, Niederalkoxycarbonyl, Carbamoyl, Sulfamoyl, Mono- oder Di-niederalkyl-(carbamoyl oder -sulfamoyl) bedeutet, $C_nH_{2n}$ für Niederalkylen steht, welches die zwei Stickstoffatome durch 2 oder 3 Kohlenstoffatome trennt, und $R_3$ für Wasserstoff, Niederalkyl, Niederalkenyl, Niederalkynyl, Niederalkanoyl, Arylniederalkyl, Niederalkoxycarbonyl, Phenylniederalkoxycarbonyl, oder (Hydroxy, Niederalkanoyloxy, Aryloxy oder Niederalkoxy)-niederalkyl steht, worin der Niederalkylrest mindestens 2 Kohlenstoffatome enthält, $R_4$ und $R_5$ unabhängig voneinander Wasserstoff, Niederalkyl, Niederalkoxy, Niederalkylthio, Halogen, Trifluormethyl, Hydroxy, Niederalkanoyloxy, Sulfamoyl, Mono- oder Diniederalkylsulfamoyl, und $R_6$ und $R_7$ Wasserstoff oder Niederalkyl bedeuten, ihren N-Oxiden, ihren Niederalkyl quaternären Derivaten und Salzen, dadurch gekennzeichnet, dass man

a) eine Verbindung der Formel III

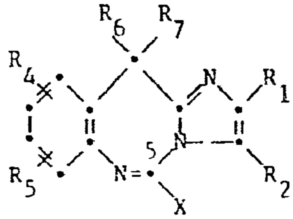

(III) ,

worin X Halogen, Niederalkoxy, Niederalkylthio, Cyanato oder Thiocyanato bedeutet, mit einer Verbindung der Formel

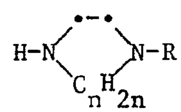

(IV)

oder einem Alkalimetallderivat davon, in welcher $R_3$ die unter Formel I angegebene Bedeutung hat, kondensiert, oder

b) eine Verbindung der Formel VI

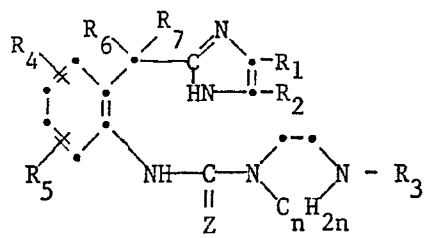

(VI)

unter entwässernden, dehydrosulfurierenden und desaminierenden Bedingungen ringschliesst, worin Z ein Sauerstoff- oder Schwefelatom oder NH bedeutet, und die anderen Symbole die oben angegebenen Bedeutungen haben, und wenn eine Verbindung der Formel I erwünscht ist, worin $R_3$ Niederalkyl bedeutet, in eine Verbindung der Formel I, worin $R_3$ Wasserstoff oder ein Alkalimetallatom ist, einen genannten Rest einführt, und/oder, wenn eine Verbindung der Formel I erwünscht ist, worin $R_3$ Niederalkyl bedeutet, eine Verbindung, in der anstelle von Niederalkyl ein Niederalkenyl- oder Niederalkynyl steht, diese zu Niederalkyl reduziert, und/oder, wenn eine Verbindung erwünscht ist, worin $R_3$ Hydroxy-niederalkyl bedeutet, eine Verbindung, worin $R_3$ Wasserstoff oder ein Alkalimetallatom bedeutet, mit entsprechenden Oxiranen oder mit reaktionsfähigen Estern von mono-veresterten Niederalkandiolen umsetzt, und/oder, wenn ein Verbindung erwünscht ist, worin $R_3$ Hydroxy-niederalkyl bedeutet, eine erhaltene Verbindung, worin $R_3$ für Wasserstoff steht, zuerst mit reaktionsfähigen Derivaten von entsprechenden Glykolen, Glykolsäuren oder Dicarbonsäuren umsetzt und die erhaltenen Verbindungen zu solchen, in welchen $R_3$ Hydroxy-niederalkyl bedeutet hydrolysiert oder reduziert, und oder, wenn

eine Verbindung erwünscht ist, worin $R_3$ Niederalkoxycarbonyl bedeutet, in einem Endprodukt, worin $R_3$ Niederalkyl bedeutet, dieses in eine Niederalkoxycarbonylgruppe überführt, und/oder, wenn eine Verbindung erwünscht ist, worin $R_3$ einen im Anspruch 1 definierten Acylrest bedeutet, eine erhaltene Verbindung, worin $R_3$ für Wasserstoff steht, acyliert, und/oder, wenn eine Verbindung erwünscht ist, worin $R_3$ Wasserstoff bedeutet, eine erhaltene Verbindung, worin $R_3$ einen Acylrest bedeutet, hydrolysiert, und/oder, wenn eine Verbindung erwünscht ist, worin $R_3$ Methyl bedeutet, in einem erhaltenen Produkt, worin $R_3$ Niederalkoxycarbonyl oder Phenylniederalkoxycarbonyl bedeutet, diese Reste zu Methyl reduziert, und/oder, wenn eine Verbindung erwünscht ist, worin $R_1$ und/oder $R_2$ Halogen bedeutet, ein erhaltenes Produkt, worin $R_1$ und/oder $R_2$ Wasserstoff bedeutet, halogeniert, und/oder, wenn eine Verbindung erwünscht ist, worin $R_1$ und/oder $R_2$ Carboxy, Carboniederalkoxy oder ein im Anspruch 1 genannter Carbamoylrest ist, eine erhaltene Verbindung, worin $R_1$ und/oder $R_2$ Wasserstoff bedeutet, mit einem Trihalogenacetyl-halogenid umsetzt und nachfolgend mit einem Alkalimetall-niederalkoxyd, -hydroxyd oder -amid behandelt, und/oder, wenn eine Verbindung erwünscht ist, worin $R_1$ und/oder $R_2$ Sulfamoyl, Mono- oder Di-niederalkylsulfamoyl bedeutet, ein Produkt, worin $R_1$ und/oder $R_2$ Wasserstoff bedeutet, mit einer Halogensulfonsäure umsetzt und nachfolgend mit Ammoniak, einem Mono- oder Di-niederalkylamin behandelt, und/oder, wenn eine Verbindung erwünscht ist, worin $R_1$ und/oder $R_2$ Cyan bedeutet, ein Produkt, worin $R_1$ und/oder $R_2$ für Carbamoyl steht, dehydratisiert, und/oder, wenn eine Verbindung erwünscht ist, worin $R_1$ und/oder $R_2$ Carboxy ist, ein Produkt, worin $R_1$ und/oder $R_2$ Cyan, Carbalkoxy oder Carbamoyl ist, hydrolysiert, und/oder, wenn ein N-Oxid erwünscht ist, ein Produkt worin $R_3$ sich von Wasserstoff unterscheidet, oxidiert, und/oder, wenn ein quaternäres Niederalkylderivat erwünscht ist, ein Produkt, worin $R_3$ sich von Wasserstoff unterscheidet oder $R_3$ Wasserstoff bedeutet, mit einem reaktionsfähig veresterten Niederalkanol umsetzt, und/oder, wenn erwünscht, eine erhaltene Verbindung der Formel I in eine andere Verbindung der Erfindung umwandelt, und/oder, wenn

BAD ORIGINAL

erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

2. Verfahren nach Patentanspruch 1, dadurch gekennzeichnet, dass man Verbindungen der Formel I, ihre N-Oxide, Niederalkyl quaternären Derivate und Salze, worin jedes der Symbole $R_1$ und $R_2$ Wasserstoff, Niederalkyl, Cyan, Carboxy, Niederalkoxycarbonyl oder Carbamoyl, und n die ganze Zahl 2-4 bedeuten, $R_3$ für Wasserstoff, Niederalkyl, Niederalkoxycarbonyl oder Hydroxy-niederalkyl mit 2-4 Kohlenstoffatomen im Niederalkylrest steht, $R_4$ Wasserstoff, Niederalkyl, Niederalkoxy, Niederalkylthio, Halogen- oder Trifluormethyl bedeutet, und $R_5$ für Wasserstoff steht, und $R_6$ und $R_7$ die Bedeutung von Wasserstoff oder Niederalkyl haben, herstellt.

3. Verfahren nach Patentanspruch 1, dadurch gekennzeichnet, dass man Verbindungen der Formel I, ihre N-Oxide, Niederalkyl quaternären Derivate und Salze, worin jedes der Symbole $R_1$ und $R_2$ Wasserstoff, Methyl, Aethyl, Cyano, Carboxy, Niederalkoxycarbonyl mit 1-3 Kohlenstoffatomen im Niederalkoxyrest oder Carbamoyl bedeutet, n eine ganze Zahl 2 oder 3 bedeutet, $R_3$ für Wasserstoff, Niederalkyl mit 1-3 Kohlenstoffatomen, Niederalkoxycarbonyl mit 1-3 Kohlenstoffatomen im Niederalkoxyrest, Hydroxyäthyl oder Hydroxypropyl steht, $R_4$ Wasserstoff, Methyl, Methoxy, Methylthio, Chlor oder Trifluormethyl, $R_5$ Wasserstoff und $R_6$ und $R_7$ Wasserstoff oder Methyl bedeuten, herstellt.

4. Verfahren nach Patentanspruch 1, dadurch gekennzeichnet, dass man Verbindungen der Formel II

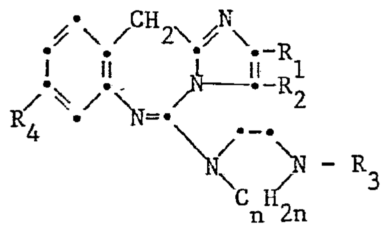

(II),

worin jedes der Symbole $R_1$ und $R_2$ unabhängig voneinander Wasserstoff oder Niederalkyl bedeutet, $R_3$ für Wasserstoff, Niederalkyl oder Hydroxy-niederalkyl steht, worin Hydroxy vom Stickstoffatom durch mindestens 2 Kohlenstoffatome getrennt ist, $R_4$ Wasserstoff, Niederalkyl, Niederalkoxy, Niederalkylthio, Halogen oder Trifluormethyl bedeutet, $C_nH_{2n}$ für Aethylen oder Propylen steht, ihre N-Oxide und Salze herstellt.

5. Verfahren nach Patentanspruch 1, dadurch gekennzeichnet, dass man Verbindungen der Formel II, worin jedes der Symbole $R_1$ und $R_2$ unabhängig voneinander Wasserstoff oder Methyl, $R_3$ Wasserstoff, Methyl, Aethyl, Propyl, 2-Hydroxyäthyl oder 3-Hydroxy-propyl bedeuten, und $R_4$ für Wasserstoff, Methyl, Methoxy, Fluor, Chlor oder Trifluormethyl steht, $C_nH_{2n}$ Aethylen oder Propylen bedeutet, ihre N-Oxide und therapeutisch verwendbaren Salze herstellt.

6. Verfahren nach Patentanspruch 1, dadurch gekennzeichnet, dass man Verbindungen der Formel II, worin $R_1$ und $R_2$ unabhängig voneinander Wasserstoff oder Methyl, und $R_3$ Wasserstoff, Methyl, Aethyl, Propyl, 2-Hydroxyäthyl bedeuten, und $R_4$ für Wasserstoff, Methyl, Fluor, Chlor oder Trifluormethyl steht und $C_nH_{2n}$ Aethylen bedeutet, und ihre therapeutisch verwenbaren Salze herstellt.

7. Verfahren nach Patentanspruch 1, dadurch gekennzeichnet, dass man 5-(4-Methyl-1-piperazinyl)-11H-imidazo[1,2-c][1,3]benzodiazepin und seine therapeutisch verwendbaren Salze herstellt.

BAD ORIGINAL

8. Verfahren nach Patentanspruch 1, dadurch gekennzeichnet, dass man 5-[4-(2-Hydroxyäthyl)-piperazinyl]-11H-imidazo[1,2-c][1,3]benzodiazepin und seine therapeutisch verwendbaren Salze herstellt.

9. Verfahren nach Patentanspruch 1, dadurch gekennzeichnet, dass man 5-(4-Aethoxycarbonyl-1-piperazinyl)-11H-imidazo[1,2-c][1,3]benzodiazepin und seine therapeutisch verwendbaren Salze herstellt.

10. Verfahren zur Herstellung von Verbindungen der Formel I, worin $R_1$, $R_2$, $R_3$, $R_4$, $R_5$, $R_6$, $R_7$ und $C_nH_{2n}$ die in Anspruch 1 erwähnten Bedeutungen haben, ihren N-Oxiden, ihren Niederalkyl quaternären Derivaten und Salzen, dadurch gekennzeichnet, dass man eine Verbindung der Formel III

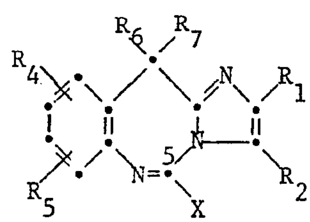

(III),

worin X eine zusammen mit Wasserstoff oder einem Alkalimetall abspaltbare Gruppe bedeutet und die anderen Symbole die unter der Formel I angegebenen Bedeutungen haben, mit einer Verbindung der Formel IV

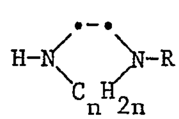

(IV)

oder einem Alkalimetallderivat davon, in welcher $R_3$ die unter der Formel I angegebene Bedeutung hat, kondensiert, und, wenn eine Verbindung der Formel I erwünscht ist, worin $R_3$ Niederalkyl bedeutet, in eine Verbindung der Formel I, worin $R_3$ Wasserstoff oder ein Alkalimetallatom ist, einen genannten Rest einführt, und/oder, wenn eine Verbindung der Formel I erwünscht ist, worin $R_3$ Niederalkyl bedeutet, eine Verbindung, in der anstelle von Niederalkyl ein Niederalkenyl- oder Niederalkynyl steht, diese zu Niederalkyl reduziert, und/oder, wenn eine Verbindung erwünscht ist, worin $R_3$ Hydroxy-niederalkyl

bedeutet, eine Verbindung, worin $R_3$ Wasserstoff oder ein Alkalimetallatom bedeutet, mit entsprechenden Oxiranen oder mit reaktionsfähigen Estern von mono-veresterten Niederalkandiolen umsetzt, und/oder, wenn eine Verbindung erwünscht ist, worin $R_3$ Hydroxy-niederalkyl bedeutet, eine erhaltene Verbindung, worin $R_3$ für Wasserstoff steht, zuerst mit reaktionsfähigen Derivaten von entsprechenden Glykolen, Glykolsäuren oder Dicarbonsäuren umsetzt und die erhaltenen Verbindungen zu solchen, in welchen $R_3$ Hydroxyniederalkyl bedeutet hydrolysiert oder reduziert, und/oder, wenn eine Verbindung erwünscht ist, worin $R_3$ Niederalkoxycarbonyl bedeutet, in einem Endprodukt, worin $R_3$ Niederalkyl bedeutet, dieses in eine Niederalkoxycarbonylgruppe überführt, und/oder, wenn eine Verbindung erwünscht ist, worin $R_3$ einen im Anspruch 1 definierten Acylrest bedeutet, eine erhaltene Verbindung, worin $R_3$ für Wasserstoff steht, acyliert, und/oder, wenn eine Verbindung erwünscht ist, worin $R_3$ Wasserstoff bedeutet, eine erhaltene Verbindung, worin $R_3$ einen Acylrest bedeutet, hydrolysiert, und/oder, wenn eine Verbindung erwünscht ist, worin $R_3$ Methyl bedeutet, in einem erhaltenen Produkt, worin $R_3$ Niederalkoxycarbonyl oder Phenylniederalkoxycarbonyl bedeutet, diese Reste zu Methyl reduziert, und/oder, wenn eine Verbindung erwünscht ist, worin $R_1$ und/oder $R_2$ Halogen bedeutet, ein erhaltenes Produkt, worin $R_1$ und/oder $R_2$ Wasserstoff bedeutet, halogeniert, und/oder, wenn eine Verbindung erwünscht ist, worin $R_1$ und/oder $R_2$ Carboxy, Carboniederalkoxy oder ein im Anspruch 1 genannter Carbamoylrest ist, eine erhaltene Verbindung, worin $R_1$ und/oder $R_2$ Wasserstoff bedeutet, mit einem Trihalogenacetylhalogenid umsetzt und nachfolgend mit einem Alkalimetall-niederalkoxyd, -hydroxyd oder -amid behandelt, und/oder, wenn eine Verbindung erwünscht ist, worin $R_1$ und/oder $R_2$ Sulfamoyl, Mono- oder Di-niederalkylsulfamoyl bedeutet, ein Produkt, worin $R_1$ und/oder $R_2$ Wasserstoff bedeutet, mit einer Halogensulfonsäure umsetzt und nachfolgend mit Ammoniak, einem Mono- oder Diniederalkylamin behandelt, und/oder, wenn eine Verbindung erwünscht ist, worin $R_1$ und/oder $R_2$ Cyan bedeutet, ein Produkt, worin $R_1$ und/oder $R_2$ für Carbamoyl steht, dehydratisiert, und/oder, wenn eine Verbindung erwünscht ist, worin $R_1$ und/oder $R_2$

Carboxy ist, ein Produkt, worin $R_1$ und/oder $R_2$ Cyan, Carbalkoxy oder Carbamoyl ist, hydrolysiert, und/oder, wenn ein N-Oxid erwünscht ist, ein Produkt, worin $R_3$ sich von Wasserstoff unterscheidet, oxidiert, und/oder, wenn ein quaternäres Niederalkylderivat erwünscht ist, ein Produkt, worin $R_3$ sich von Wasserstoff unterscheidet oder $R_3$ Wasserstoff bedeutet, mit einem reaktionsfähig veresterten Niederalkanol umsetzt, und/oder, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

11. Verfahren nach Patentanspruch 10, dadurch gekennzeichnet, dass man Verbindungen der Formel I, ihre N-Oxide, Niederalkyl quaternären Derivate und Salze, worin $R_1$-$R_7$ und $C_nH_{2n}$ die im Anspruch 2 angegebenen Bedeutungen haben, herstellt.

12. Verfahren nach Patentanspruch 10, dadurch gekennzeichnet, dass man Verbindungen der Formel I, ihre N-Oxide, Niederalkyl quaternären Derivate und Salze, worin $R_1$-$R_7$ und $C_nH_{2n}$ die im Anspruch 3 angegebenen Bedeutungen haben, herstellt.

13. Verfahren nach Patentanspruch 10, dadurch gekennzeichnet, dass man Verbindungen der Formel II

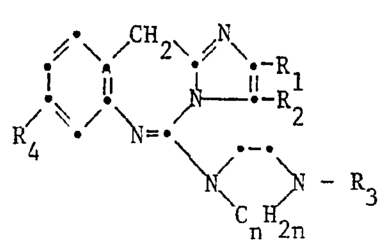

(II),

worin jedes der Symbole $R_1$ und $R_2$ unabhängig voneinander Wasserstoff oder Niederalkyl bedeutet, $R_3$ für Wasserstoff, Niederalkyl oder Hydroxy-niederalkyl steht, worin Hydroxy vom Stickstoffatom durch mindestens 2 Kohlenstoffatome getrennt ist, $R_4$ Wasserstoff, Niederalkyl, Niederalkoxy, Niederalkylthio, Halogen oder Trifluormethyl bedeutet, $C_nH_{2n}$ für Aethylen oder Propylen steht, ihre N-Oxide und Salze herstellt.

14. Verfahren nach Patentanspruch 10, dadurch gekennzeichnet, dass man Verbindungen der Formel II, worin jedes der Symbole $R_1$ und $R_2$ unabhängig voneinander Wasserstoff oder Methyl, $R_3$ Wasserstoff, Methyl, Aethyl, Propyl, 2-Hydroxyäthyl oder 3-Hydroxy-propyl bedeuten, und $R_4$ für Wasserstoff, Methyl, Methoxy, Fluor, Chlor oder Trifluormethyl steht, $C_nH_{2n}$ Aethylen oder Propylen bedeutet, ihre N-Oxide und therapeutisch verwendbaren Salze herstellt.

15. Verfahren nach Patentanspruch 10, dadurch gekennzeichnet, dass man Verbindungen der Formel II, worin $R_1$ und $R_2$ unabhängig voneinander Wasserstoff oder Methyl, und $R_3$ Wasserstoff, Methyl, Aethyl, Propyl, 2-Hydroxyäthyl bedeuten, und $R_4$ Wasserstoff, Methyl, Fluor, Chlor oder Trifluormethyl steht und $C_nH_{2n}$ insbesondere Aethylen bedeutet, und ihre therapeutisch verwendbaren Salze herstellt.

16. Verfahren nach Patentanspruch 10, dadurch gekennzeichnet, dass man 5-(4-Methyl-1-piperazinyl)-11H-imidazo[1,2-c][1,3]benzodiazepin und seine therapeutisch verwendbaren Salze herstellt.

17. Verfahren zur Herstellung eines pharmazeutischen Präparates, gekennzeichnet durch die Verarbeitung eines erfindungsgemässen Wirkstoffes nach einem der Ansprüche 1 bis 9, mit einem pharmazeutischen Trägermaterial.

18. Verfahren zur Herstellung eines pharmazeutischen Präparates, gekennzeichnet durch die Verarbeitung eines erfindungsgemässen Wirkstoffes nach einem der Ansprüche 9-16 mit einem pharmazeutischen Trägermaterial.